(19) Europäisches Patentamt
European Patent Office
Office européen des brevets

(11) **EP 4 015 614 A1**

(12) **EUROPEAN PATENT APPLICATION**

(43) Date of publication:
**22.06.2022 Bulletin 2022/25**

(21) Application number: **20214285.7**

(22) Date of filing: **15.12.2020**

(51) International Patent Classification (IPC):
**C12M 1/00** $^{(2006.01)}$ **C12M 1/26** $^{(2006.01)}$

(52) Cooperative Patent Classification (CPC):
**C12M 29/10; C12M 29/18; C12M 33/10; C12M 47/10**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Alfa Laval Corporate AB**
**221 00 Lund (SE)**

(72) Inventors:
• **KÖNIGSSON, Staffan**
  **SE-147 31 TUMBA (SE)**
• **THORWID, Peter**
  **SE-172 66 SUNDBYBERG (SE)**

(74) Representative: **Alfa Laval Attorneys**
**Alfa Laval Corporate AB**
**Patent Department**
**P.O. Box 73**
**221 00 Lund (SE)**

(54) **METHOD FOR OPERATING A BIOPROCESSING SYSTEM AND A BIOPROCESSING SYSTEM**

(57) The disclosure concerns a method (100) for operating a bioprocessing system, comprising steps of: - producing (102) a cell culture mixture in a fermentor (4), - conducting (104) a flow of cell culture mixture from the fermentor (4) into an interior of a centrifugal separator (6) comprising a surface enlarging insert, simultaneously with the step of producing (102) the cell culture mixture, and - returning (106) continuously from the interior of the centrifugal separator (6) a flow of liquid to the fermentor (4), simultaneously with the steps of producing (102) the cell culture mixture and conducting (104) the flow of cell culture mixture.

Fig. 1

**Description**

TECHNICAL FIELD

The invention relates to methods for operating a bioprocessing system and to bioprocessing systems. The invention further relates to a computer program and to a computer program product.

BACKGROUND

WO 2017/025210 discloses a disposable bioprocess system for continuous processing of micro-organisms in the pharmaceutical industry integrating a single use bioreactor, a single use pump, and a single use cross flow filter. The system provides for perfusion mode processes.

As opposed to batch operation, an operation method to which no fresh media is added and no used media and/or liquid is removed, and fed-batch operation wherein the process starts with a small media volume to which fresh medium is added and no liquid is removed until after termination of the process, perfusion mode operation relates to operation methods where the media is sequentially exchanged, fresh nutrients are sequentially added, and product i.e. used media is removed throughout the culture period. Perfusion mode operation typically may last 4 - 8 times longer than batch operation and 2 - 4 times longer than fed-batch operation.

Many common methods of producing biological proteins in recombinant mammalian and microbial cells rely on fed-batch cultures, wherein cells are grown to high cell densities and then typically exposed to an induction medium or inducer to trigger the production of proteins. If the desired proteins are secreted out of the cells, it is more profitable to switch from a fed-batch culture to a continuous perfusion culture, which can maintain high cell density and high productivity over a much longer duration of culture. During continuous perfusion cultures, live and productive cells are retained or recycled back to the bioreactor while the secreted proteins are continuously harvested from the bioreactor for downstream purification processes.

Some key advantages of continuous perfusion cultures over fed-batch cultures are: (1) the secreted protein products are continuously removed from the bioreactor, without subjecting these products to potential degradation by proteolytic and/or glycolytic enzymes released into the culture medium from dead cells; (2) live and productive cells are retained or recycled back to achieve high cell densities in continuous perfusion bioreactors, where they continue to produce valuable proteins inside the controlled bioreactor environment for much longer culture duration, rather than being killed and removed from the bioreactor at the end of each fed-batch culture; (3) the perfusion bioreactor environment can be maintained much closer to steady state conditions (thereby maintaining a more consistent product quality by design) with the continuous addition of fresh nutrient media and removal of waste products along with the harvested protein products, unlike the dynamically changing concentrations of nutrients and waste products in fed-batch culture.

A cross flow filter (CFF), or similar filter such as alternating tangential flow (ATF) filter, of the membrane type is commonly utilised for performing perfusion mode processes. A flow of the cell culture herein also referred to as cell culture mixture, from the fermentor passes the filter. Part of the cell culture mixture flows through the membrane and is relieved of the cells of the cell culture. This part forms a filtrate i.e. product which is collected for downstream processing. The remaining portion of the cell culture mixture, a retentate, which contains cells is conducted from the membrane back to the fermentor.

Filter technology has its disadvantages, mainly that the permeability of the membrane gradually decreases during use thereof. This aspect is particularly relevant in a bioprocessing system operating in perfusion mode, which may be performed over several weeks or months. Thus, either the membrane or filter is to be regularly replaced or an unfeasible large membrane area has to be provided in order to ensure proper functionality of the bioprocessing system over a longer period of time.

SUMMARY

It would be advantageous to achieve a method for operating a bioprocessing system and/or a bioprocessing system overcoming, or at least alleviating, the above-mentioned drawback. In particular, it would be desirable to enable perfusion mode operation of a compact bioprocessing system over longer periods of time. To address inter alia this concern, at least one of a method for operating a bioprocessing system and a bioprocessing system having the features defined in the independent claims is provided.

According to an aspect there is provided a method for operating a bioprocessing system, comprising steps of:

- producing a cell culture mixture in a fermentor,
- conducting a flow of cell culture mixture from the fermentor into an interior of a centrifugal separator comprising a surface enlarging insert, simultaneously with the step of producing the cell culture mixture, and

- returning continuously from the interior of the centrifugal separator a flow of liquid to the fermentor, simultaneously with the steps of producing the cell culture mixture and conducting the flow of cell culture mixture.

According to a further aspect, there is provided a bioprocessing system comprising a fermentor for producing therein a cell culture mixture, a centrifugal separator comprising a surface enlarging insert, a receiving container, a conduit system interconnecting the fermentor, the centrifugal separator, and the receiving container, flow control equipment arranged in the conduit system, and a control system configured for controlling the centrifugal separator and at least part of the flow control equipment. The control system is configured to:

- conduct a flow of cell culture mixture from the fermentor into an interior of the centrifugal separator, and simultaneously,
- continuously return from the interior of the centrifugal separator a flow of liquid to the fermentor.

Since each of the method and bioprocessing system provides the continuous return of a flow of liquid from the interior of the centrifugal separator comprising a surface enlarging insert to the fermentor, perfusion mode operation of a bioprocessing system which comprises a centrifugal separator comprising a surface enlarging insert is enabled. More specifically, in the interior of the centrifugal separator comprising a surface enlarging insert there is a volume into which cells of the cell culture mixture flow, providing continuous return from the interior of the centrifugal separator to the fermentor ensures that stagnant conditions in this volume, i.e. in the interior of the centrifugal separator comprising a surface enlarging insert are avoided. Stagnant cells culture mixture or cell containing portions thereof would deteriorate and degrade the production of cell culture mixture in the fermentor if returned to the fermentor. Thus, each of the method and bioprocessing system are adapted for perfusion mode operation by the continuous return of a flow of liquid from the interior of the centrifugal separator comprising a surface enlarging insert to the fermentor.

It has been found that a centrifugal separator with a surface enlarging insert may be used not only during harvesting of a produced biomolecule at a final stage of a bioprocess, but also continuously during several stages of a bioprocess operating in perfusion mode for e.g., removal of a medium containing the sought-after substance and cell debris without damaging live cells, which in turn continuously may be returned to the fermentor.

Moreover, advantages of the centrifugal separator comprising a surface enlarging insert thus, can be utilised in the context of perfusion mode operation of the bioprocessing system, such as space efficiency; efficient separation of cells; gentle treatment of the cells being separated by continuously being transported out of the centrifugal separator with a separated heavy phase in comparison with filter technology wherein the cells are amassed on a filter surface over shorter or longer periods of time; self-cleaning functionality; continuous operation over long periods of time without replacement of parts as may be the case in the context of filter technology; etc.

Further, the use of a centrifugal separator comprising a surface enlarging insert provides for an efficient fractionation, or classification, of the cell culture mixture when separated in the centrifugal separator. That is, the largest particle size to be separated with a separated light phase by the centrifugal separator, the so-called critical particle, may be efficiently controlled by controlling the rotational speed of the rotor of the centrifugal separator and the flow of cell culture mixture into the centrifugal separator. Thus, the critical particle may be set to include undamaged whole cells with the heavy phase and to exclude them from the light phase.

Cell debris and other debris (which debris is smaller than whole cells) flows with both the light and heavy phases. Accordingly, with separated light phase not being returned to the fermentor, also debris is removed from the fermentor, which debris is not desirable in the fermentor.

In comparison with filter technology, utilising the centrifugal separator comprising a surface enhancing insert, firstly, the critical particle is easily adjusted and secondly, and more importantly, the critical particle may be maintained over the entire use period of the centrifugal separator. Filter technology suffers from its permeability decreasing as particles settle on the filter membrane, which entails that over time a larger amount of smaller than intended particles remain in the retentate and are returned to the fermentor.

The cell culture mixture is produced in the fermentor of the bioprocessing system. The term "fermentor" is used herein is intended to relate to any container wherein a cell culture mixture may be produced and may alternatively be referred to as a bioreactor or reactor. The fermentor may alternatively be referred to as fermenter. The fermentor may for instance be a so-called Stirred-Tank-Reactor" (STR) integrating an aeration device and agitation or mixing device(s) for forced exchange of nutrient and gas with the cells within the cell culture mixture. Herein the term "bioprocess" is used for the process/es being performed in the bioprocessing system.

Fermentation and/or cultivation takes place in the fermentor during the step of producing the cell culture mixture therein. The terms "fermentation" and "cultivation" as used herein refers to hosting of micro-organisms or mammalian cells, such as living single-celled organisms, prokaryotes, eucaryotic cells, human cells, CHO, and bacteria for industrial purposes in a fermentor expressing an extracellular biomolecule. Fermentation / cultivation produces the cells of the cell culture mixture and the expressed biomolecule. The fermentation takes place over a culture period. The culture period may extend over several week during a large portion of which the product containing the extracellular biomolecule is produced, due to the perfusion mode operation of the bioprocessing system. The fermentation may for example be for expression of an extracellular biomolecule, such as an antibody or a protein, from a mammalian cell culture mixture. Accordingly, the product contains the extracellular biomolecule. For instance, the cell culture mixture may comprise cells such as CHO (Chinese Hamster Ovary) cells and a growth medium composed to promote growth of the cells. CHO being a micro-organism and mammalian cell line very popular as expression platform of proteins for the pharma industry.

Further, in the fermentor, conditions for promoting growth of the cells is provided, e.g. by the addition of growth medium, oxygen, and maintenance of a suitable temperature range.

The growth medium also referred to as culture medium is a liquid, the constituents of which promote growth of cells in the cell culture mixture and contains inter alia water, glucose, salts, and a source of amino acids. A gas such as clean air or oxygen is added to the growth medium e.g. in the fermentor.

The term "cell culture mixture" as used herein means the non-separated liquid content, the cultivation soup in a fermentor or bioreactor consisting of cells, debris, microorganisms, media with nutrients, waste, product, etc. During e.g. a first time period, the cell culture mixture may be separated into a light phase and a heavy phase in the centrifugal separator comprising a surface enlarging insert, whereby the cell density of the heavy phase increases over that of the entering cell culture mixture and as such is returned as the above mentioned flow of liquid to the fermentor and remixed with the cell culture mixture in the fermentor.

The centrifugal separator comprising a surface enlarging insert may herein alternatively be referred to as centrifugal separator. It comprises a rotor which defines a separation space. Inside the rotor, in the separation space, the surface enlarging insert is arranged. The surface enlarging insert may comprise e.g. axially arranged separation members or frustoconical separation discs arranged in a disc stack. An inlet is provided for conducting the cell culture mixture into the separation space, i.e. into the interior of the centrifugal separator. Further, an outlet is provided for separated light phase and an outlet for separated heavy phase.

When separation takes place in the centrifugal separator, e.g. during the first time period, the rotor is rotated at a speed such that the cell culture mixture is separated into the light phase and the heavy phase.

Suitably, the outlet for separated heavy phase from the centrifugal separator is provided concentrically with or close to a rotational axis of the rotor. In this manner, the cells in the separated heavy phase are subjected to low shear forces and accordingly, are gently treated as the heavy phase leaves the rotor of the centrifugal separator and is led back to the fermentor. Similarly, the inlet for the cell culture mixture of the centrifugal separator may be provided concentrically with or close to the rotational axis of the rotor.

The conduit system may comprise one or more of tubes, hoses, pipes, etc. configured for conducting liquid such as the cell culture mixture, separated light phase, and separated heavy phase within the bioprocessing system, and/or to and/or from external components connected to the bioprocessing system.

The flow control equipment may comprise one or more pumps and valves configured for pumping and directing liquid through the bioprocessing system, and/or to and/or from external components connected to the bioprocessing system.

The term "control system" as used herein refers to a Programmable Logic Control (PLC), a Personal Computer (PC), an Embedded Computer (EC) or similar device integrating a Central-Processing-Unit (CPU) electronic device with calculating power. The control system has various Input & Output (I/O) for the centrifugal separator and the flow control equipment. The control system integrates software, algorithms, process recipe in build-in memory for controlling the centrifugal separator and the flow control equipment. Optionally, the control system may also control aspects of the fermentor, e.g. for managing, analysis of the cultivation or fermentation process for ongoing process alignment according to the process recipe. The control system may correspond with a variety of sensors and actuators in order to alter the process parameters.

The term "product" is herein used for the media being expected to be produced or generated in the bioprocessing system. The light phase being separated in the centrifugal separator comprising a surface enlarging insert may form the product or may alternatively contain the product. The product may be subjected to downstream processing in order to extract therefrom a sought-after substance, such as an extracellular biomolecule, e.g. an antibody or a protein. That is, the product may be an intermediate result in a larger context of producing an end produce.

The term "cell/cells" as used herein is typically divided into: 1. living single-celled organisms, microbes such as; fungus, algae, moss, plankton, yeast, protozoa, eukaryotes, archaea, micro animals, extremophiles and plant cells or the like - 2. adherent or semi adherent or suspended living cells such as animal cells, insect cells, mammalian cells, human cells, stem cells - 3. prokaryotes and a variety of bacteria such as E.coli or the like. Most of the above may be genetically modified to provide a particular sought-after substance.

The term "perfusion" mode operation as used herein refers to the operation method or principle for a fermentor where the media is sequentially exchanged, fresh nutrients sequentially added, used media / product removed throughout a major portion of the culture period. The micro-organisms and mammalian cells retained in the perfusion mode operation may last typically 4 - 8 times longer than in batch operation and 2 - 4 times longer than in fed-batch operation. Mentioned purely as examples, perfusion mode operation may last a number of days, one or more weeks, or even one or more months.

Perfusion mode operation supports efficient facility utilization by offering the possibility of using a smaller bioreactor /fermentor. Compared with batch or fed-batch processes, perfusion mode operation allows the cells of the cell culture mixture to stay in an exponential growth phase for an extended time and reach higher viable cell densities.

Moreover, the perfusion mode operation entails that the continuous return of the flow of liquid from the centrifugal separator to the fermentor and that the step of returning continuously from the interior of the centrifugal separator the flow of liquid to the fermentor, may take place over more than 90%, such as more than 95 %, of a duration of the entire culture period.

The term "single-use" as used herein refers to a device designed for use only once, such as for one full culture period of a fermentation process operated in perfusion mode, and to be disposed after use. The device is typically delivered sterilized and ready to use, such as a single-use portion of the centrifugal separator and a single-use fermentor.

According to embodiments, the method may comprise during a first time period a step of:

- separating the flow of cell culture mixture into a light phase and a heavy phase in the centrifugal separator, and wherein the step of returning continuously from the interior of the centrifugal separator a flow of liquid to the fermentor may comprise during the first time period:
- returning the heavy phase separated in the centrifugal separator to the fermentor as the flow of liquid to the fermentor. In this manner, the centrifugal separator comprising a surface enlarging insert may be utilised during the first time period to separate the cell culture mixture from the fermentor into the light phase and the heavy phase. More specifically, during the first time period, the light phase containing the product produced in the bioprocessing system may be separated while the cells of the cell culture mixture are returned to the fermentor with the heavy phase. Thus, there may be provided for perfusion mode operation of a bioprocessing system which comprises a centrifugal separator with a surface enlarging insert.

The light phase separated in the centrifugal separator is a liquid. The heavy phase separated in the centrifugal separator is a suspension comprising cells of the cell culture mixture suspended in the same liquid as that forming the light phase. The separated light phase may contain an extracellular biomolecule that has been expressed by the cells during fermentation. During the first time period, the separated light phase may comprise debris. Such debris may be of smaller size than the cells of the cell culture mixture. Namely, the cells may be separated with the heavy phase. Also, some of smaller size particles than the cells of the cell culture mixture may be separated with the heavy phase.

During the first time period, the step of producing the cell culture mixture in the fermentor and the step of conducting the flow of cell culture mixture from the fermentor into the interior of the centrifugal separator are performed.

According to embodiments, in the step of separating the flow of cell culture mixture into a light phase and a heavy phase, a flow of the separated light phase may form a maximum of 5 %V/V (volume %) of the flow of cell culture mixture. In this manner, an appropriately small amount of light phase / product may be drawn from the cell culture mixture in the perfusion mode of operation of the bioprocessing system.

According to embodiments, the method may comprise during the first time period a step of:

- conducting the light phase separated in the centrifugal separator to a receiving container. In this manner, the light phase / product may be collected in a convenient manner for further processing.

Fresh growth medium may be supplied to the fermentor based on the amount of separated light phase that is conducted to the receiving container. In this manner, the light phase that is being removed from the fermentor may be replace with growth medium, which in turn promotes the growing of cells and their production of the sought-after substance.

According to embodiments, preceding the first time period, the method may comprise a step of:

- priming the centrifugal separator with a liquid other than cell culture mixture, such as a growth medium. In this manner, the centrifugal separator and the bioprocessing system may be prepared for perfusion mode operation. Due to the priming of the centrifugal separator with the liquid other than cell culture mixture, the cell culture mixture from the fermentor may flow evenly into the interior of the centrifugal separator at the onset of the first time period.

According to embodiments, the step of returning continuously from the interior of the centrifugal separator a flow of liquid to the fermentor may comprise during a second time period a step of:

- returning the flow of cell culture mixture from the interior of the centrifugal separator unseparated to the fermentor as the flow of liquid to the fermentor. In this manner, there is provided for different operation of the centrifugal separator during the first and second time periods, respectively. The centrifugal separator comprising a surface enlarging insert may be utilised during the first time period to separate the cell culture mixture from the fermentor into the light phase and the heavy phase while during the second time period, the cell culture mixture may flow through the centrifugal separator back to the fermentor. Thus, there is provided for perfusion mode operation of a bioprocessing system which comprises a centrifugal separator comprising a surface enlarging insert when its separation capacity exceeds separation of the cell culture mixture at a relevant flow rate from the fermentor.

More specifically, during the first time period, the light phase may be separated containing the product produced in the bioprocessing system while the cells of the cell culture mixture may be returned to the fermentor with the heavy phase. During the second time period, the centrifugal separator thus, may be inoperable and in standby in the sense that no separation of the light and heavy phases takes place therein. Instead, the cell culture mixture only flows through the interior of the centrifugal separator back to the fermentor. Therefore, during the second time period, the cell culture mixture within the centrifugal separator is continuously exchanged. Degrading of the cell culture mixture in the centrifugal separator thus, may be avoided during the second time period when no product containing light phase is drawn from the cell culture mixture.

During the second time period, no separation may take place in the centrifugal separator. The flow of cell culture mixture that is maintained through the centrifugal separator during the second period, may serve a purpose of preventing stagnant cell culture mixture, inter alia within the centrifugal separator. Such stagnant cell culture mixture might otherwise degrade the light phase and/or heavy phase separated in subsequent separation operations.

Also during the second time period, the step of producing the cell culture mixture in the fermentor and the step of conducting the flow of cell culture mixture from the fermentor into the interior of the centrifugal separator are performed.

After the second time period, a further first time period may follow and a further second time period and so on, e.g. over a large part of the entire culture period. Thus, the method and the bioprocessing system may be adapted for intermittent separation of light and heavy phases from the cell culture mixture.

In accordance with these embodiments, it has been realised by the inventors that in order to utilise a centrifugal separator with a surface enlarging insert and its advantages in a bioprocessing system that is operated in perfusion mode, separation in the centrifugal separator may have to take place intermittently. Depending on the size, and thus, capacity, of the disk stack centrifugal separator, and due to the efficient separation provided by the centrifugal separator, and depending on the size of the fermentor, the centrifugal separator cannot operate continuously over the entire culture period. The throughflow would be too great such that the separated light phase would not have the content expected from the cell culture mixture produced in the fermentor. Thus, the inventors present a method and a bioprocessing system which provides conditions for being operated intermittently without risk of degrading the separated light and heavy phases or the cell culture mixture.

According to embodiments, during the second time period during the step of returning the flow of cell culture mixture, a rotor of the centrifugal separator may be standing still or rotating at a lower rotational speed than during the first time period during the step of separating the flow of cell culture mixture, or rotating in a rotational direction opposite to a rotational direction during the first time period during the step of separating the flow of cell culture mixture. In this manner, it may be ensured that no separation or substantially no separation takes place in the centrifugal separator during the second time period. Thus, build-up of separated cells may be avoided during the second time period.

According to embodiments, during the first and second time periods, the flow of the cell culture mixture may be conducted at substantially a same flowrate, q, into the interior of the centrifugal separator. In this manner, an even flow of cell culture mixture may be maintained over the first and second time periods. Thus, an accordingly predetermined flowrate of the culture mixture may at least not be exceeded, which may ensure that the cells of the cell culture mixture are not subjected to harmful shear stress in the bioprocessing system. Moreover, one or more pumps configured for transporting the cell culture mixture and the heavy phase through the bioprocessing system may not require resetting between the first and second time periods.

According to embodiments, the method may comprise during a third time period steps of:

- stopping the step of returning continuously from the interior of the centrifugal separator a flow of liquid to the fermentor,
- conducting a flow of the cell culture mixture from the fermentor to the centrifugal separator,
- separating the flow of cell culture mixture in the centrifugal separator into a light phase and a heavy phase, and
- conducting the heavy phase separated in the step of separating to a receiving vessel. In this manner, the cell concentration of the cell culture mixture may be reduced. For instance, if the cell concentration of the cell culture mixture exceeds a limit value, during the third time period the method and bioprocessing system, thus, provide for the reduction of the cell concentration by conducting the separated heavy phase to a separate receiving vessel instead of returning the separated heavy phase to the fermentor as during the first time period.

According to embodiments, during the third time period, a rotor of the centrifugal separator may be rotating at a higher rotational speed than during the first time period. In this manner, the separated heavy phase during the third time period may have a higher concentration of particles than the separated heavy phase during the first time period. Accordingly, the reduction of cell concentration of the cell culture mixture may be efficiently performed and may also include particles smaller than whole cells.

The third time period may be followed by a first time period including the steps of separating the cell culture mixture and returning the separated heavy phase to the fermentor or by a second time period including the step of returning the unseparated cell culture mixture to the fermentor.

According to embodiments, the method may comprise during a fourth time period steps of:

- stopping the step of returning continuously from the interior of the centrifugal separator a flow of liquid to the fermentor, if not previously performed,
- conducting all of the cell culture mixture from the fermentor to the centrifugal separator,
- separating the cell culture mixture in the centrifugal separator into a light phase and a heavy phase, the light phase forming 50 - 95 %V/V of the cell culture mixture, and
- conducting the light phase separated in the centrifugal separator to a receiving container. In this manner, the fermentor may be emptied at the end of the culture period and the light phase / product may be extracted from the cell culture mixture during the fourth time period.

The ratio of how large a portion the light phase forms in comparison with the cell culture mixture depends on the cell concentration in the relevant cell culture mixture.

According to embodiments, subsequently to the third and/or fourth time period, the method may comprise steps of:

- diluting the heavy phase in the receiving vessel with a liquid to produce a diluted heavy phase,
- transferring during a fifth time period the diluted heavy phase from the receiving vessel to a centrifugal apparatus, such as the centrifugal separator,
- separating during the fifth time period the diluted heavy phase in the centrifugal apparatus into a light phase and a further heavy phase, and
- conducting the light phase to a receiving container. In this manner, a yield from the bioprocess may be increased. The heavy phase separated during one or more of the first, third, and fourth time period may be diluted with a liquid. By separating the diluted heavy phase, light phase containing the sought-after substance may be extracted which otherwise might go to waste.

According to embodiments, during the fifth period, the rotor of the centrifugal separator may be rotated at a higher rotational speed than during the first time period. In this manner, the separated heavy phase during the fifth time period may have a higher concentration of particles than the separated heavy phase during the first time period. A larger portion of light phase may be separated during the fifth time period than during the first time period.

According to embodiments, subsequently to the third, fourth, and/or fifth time period, the method may comprise during a sixth time period steps of:

- flushing the centrifugal separator with a liquid, such as a growth medium,
- running the centrifugal separator, and
- conducting at least part of the liquid via a light phase outlet of the centrifugal separator to the receiving container. In this manner, remaining product containing the sought-after substance may be flushed out of the centrifugal separator and thus, may be available for downstream processing.

According to embodiments, subsequently to any one of the fourth, fifth, and/or sixth time period, the method may comprise steps of:

- conducting the light phase collected in the receiving container to the centrifugal separator or to a centrifugal apparatus,
- separating the light phase in the centrifugal separator or the centrifugal apparatus into a light fraction and a heavy fraction, the heavy fraction comprising remaining debris from the cell culture mixture, and
- transferring the light fraction for further processing, such as extraction of an extracellular substance from the light fraction. In this manner, the light phase separated during any one of the fourth, fifth, and/or sixth time period may be cleaned from at least most of the debris that it may contain and thus, prepare the light phase for downstream processing by presenting it in the form of the separated light fraction.

According to embodiments, the centrifugal separator comprising a surface enlarging insert may be a modular centrifugal separator comprising a base unit and an exchangeable separation insert mounted in the base unit. The method may comprise a step of:

- exchanging a used exchangeable separation insert for an unused exchangeable separation insert prior to the step of conducting a flow of cell culture mixture from the fermentor into an interior of a centrifugal separator comprising a surface enlarging insert. In this manner, the centrifugal separator may be presented sterile and ready for performing its separation duty or duties in the method for operating the bioprocessing system. A single use centrifugal separator is thus, provided.

The herein discussed bioprocessing system, and in particular its control system, may be configured for performing one or more of the method steps discussed herein. In doing so, the bioprocessing system may benefit from the same or similar advantages as discussed with respect to corresponding features and/or steps of the method for operating a bioprocessing system.

According to a further aspect, there is provided a method for operating a bioprocessing system, the bioprocessing system comprising a fermentor for producing therein a liquid cell culture mixture using a growth medium, and a centrifugal separator comprising a surface enlarging insert, wherein the method comprising steps of:

- conducting continuously during a first time period a flow of the cell culture mixture from the fermentor into the interior of the centrifugal separator,
- separating continuously during the first time period the flow of the cell culture mixture in the centrifugal separator into a light phase and a heavy phase, the light phase forming a maximum of 5 %V/V of the flow of the cell culture mixture, and
- returning the heavy phase to the fermentor during the first time period simultaneously with the step of separating

According to a further aspect, there is provided a use of a centrifugal separator comprising a surface enlarging insert for separating a cell containing heavy phase from a cell culture mixture during a fermentation process operated in perfusion mode.

Above, two aspects of a method for operating a bioprocessing system are discussed, an aspect related to a bioprocessing system, and an aspect related to the use of a centrifugal separator. Each of these aspects, according to the two methods, the bioprocessing system, and the use of the centrifugal separator may utilise features of the various embodiments discussed herein. Accordingly, also features of, and advantages with, the invention discussed in the following detailed description relate to all aspects of the invention.

According to a further aspect there is provided a computer program comprising instructions which, when the program is executed by a computer, cause the computer to carry out a method according to any one of aspects and/or embodiments discussed herein.

According to a further aspect there is provided a computer-readable storage medium comprising instructions which, when executed by a computer, cause the computer to carry out a method according to any one of aspects and/or embodiments discussed herein.

Further features of, and advantages with, the invention will become apparent when studying the appended claims and the following detailed description.

BRIEF DESCRIPTION OF THE DRAWINGS

Various aspects and/or embodiments of the invention, including its particular features and advantages, will be readily understood from the example embodiments discussed in the following detailed description and the accompanying drawings, in which:

Fig. 1 illustrates embodiments of a bioprocessing system,
Fig. 2 illustrates further embodiments of a bioprocessing system,
Fig. 3 illustrates embodiments of a centrifugal separator to be utilised in a bioprocessing system,
Fig. 4 illustrates a control system according to embodiments,
Fig. 5 illustrates embodiments of a method for operating a bioprocessing system,
Fig. 6 illustrates embodiments of a computer-readable storage medium, and
Fig. 7 illustrates a diagram over particle distribution within a cell culture mixture.

DETAILED DESCRIPTION

Aspects and/or embodiments of the invention will now be described more fully. Like numbers refer to like elements throughout. Well-known functions or constructions will not necessarily be described in detail for brevity and/or clarity.

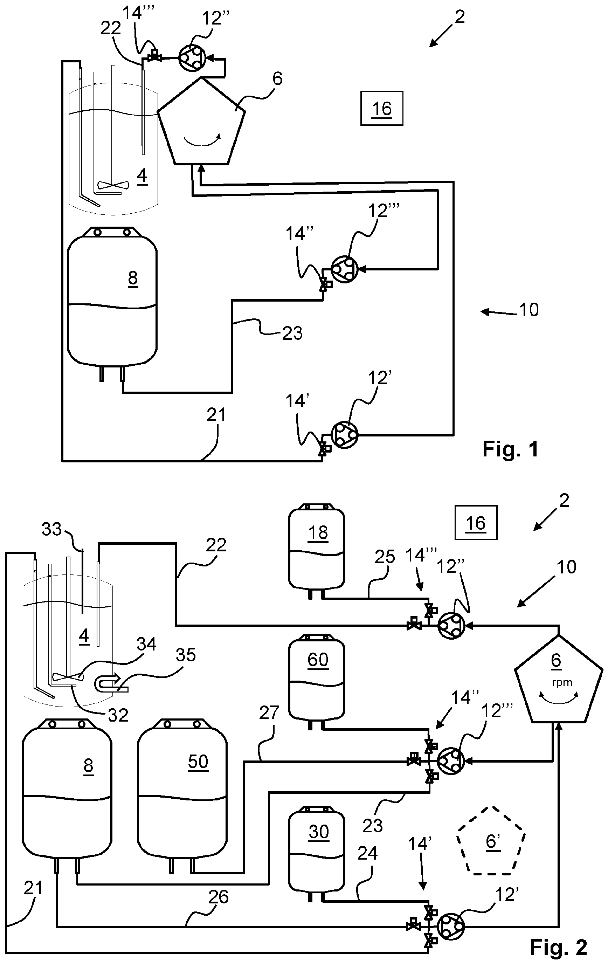

**Fig. 1** illustrates embodiments of a bioprocessing system 2.

The bioprocessing system 2 comprises a fermentor 4 for producing therein a cell culture mixture, a centrifugal separator 6 comprising a surface enlarging insert, a receiving container 8, a conduit system 10, flow control equipment 12, 14, and a control system 16.

The receiving container 8 may be configured for receiving a light phase separated in the centrifugal separator 6. The receiving container 8 may be configured for intermediate storage of the separated light phase.

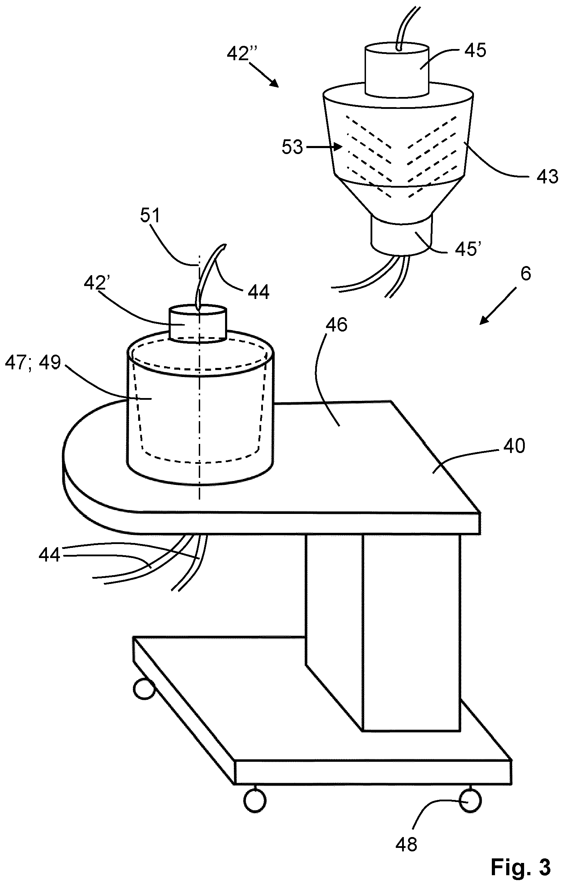

The centrifugal separator 6 may be a disc stack centrifugal separator 6 as discussed below with reference to **Fig. 3**. However, the bioprocessing system 2 in its broadest aspects is not limited to a particular kind of centrifugal separator 6 comprising a surface enlarging insert. Thus, according to some embodiments the centrifugal separator 6 may be configured for single use operation and may comprise an exchangeable separation insert. According to alternative embodiments, the centrifugal separator 6 may be configured for permanent and/or long-term use. In such embodiments, the centrifugal separator 6 may be cleaned in between culture periods.

The conduit system 10 is arranged to interconnect the fermentor 4, the centrifugal separator 6, and the receiving container 8. The flow control equipment 12, 14 is arranged in the conduit system 10. The flow control equipment may comprise one or more pumps 12 and/or one or more valves 14. The flow control equipment 12, 14 is arranged to control flow of one or more of the cell culture mixture, the heavy phase separated in the centrifugal separator 6, and the light phase separated in the centrifugal separator 6. Also, the centrifugal separator 6, may have a flow controlling function in the conduit system 10, e.g. when a rotor thereof is rotating.

The control system 16 is configured for controlling the centrifugal separator 6 and at least part of the flow control equipment 12, 14.

The system 2 is configured for perfusion mode operation utilising the centrifugal separator comprising a surface enlarging insert.

**Fig. 2** illustrates further embodiments of a bioprocessing system 2. The bioprocessing system 2 of **Fig. 2** comprises the same basic features as the system 2 of **Fig. 1** as discussed above. The bioprocessing system 2 according to the embodiments of **Figs. 1 and/or 2** and various of their components, optionally, may comprise one or more of the following listed you features:

- The bioprocessing system 2 may comprise a receiving vessel 18, and the conduit system 10 may interconnect the centrifugal separator 6 with the receiving vessel 18. The receiving vessel 18 may be configured for receiving heavy phase separated in the centrifugal separator 6. The receiving vessel 18 may be configured for storing the separated heavy phase, such as for intermediate storage of the separated heavy phase. The control system 16 configuration/s for incorporating the receiving vessel 18 in the operation of the bioprocessing system 2 is discussed below.

- The conduit system 10 may comprise a first conduit section 21 extending from the fermentor 4 to an inlet of the centrifugal separator 6, a second conduit section 22 extending from a heavy phase outlet of the centrifugal separator to the fermentor 4, a third conduit section 23 extending from a light phase outlet of the centrifugal separator 6 to the receiving container 8. In this manner, the conduit system 10 may interconnect the centrifugal separator 6, the fermentor 4, and the receiving container 8.

- The flow control equipment 16 may comprise one or more of a feed pump 12' arranged in the first conduit section 21, a heavy phase pump 12" arranged in the second conduit section 22, a light phase pump 12'" arranged in the third conduit section 23. The feed pump 12' may be utilised for transporting cell culture mixture from the fermentor 4 to the centrifugal separator 6. According to some embodiments, the feed pump 12' may also be utilised for transporting cell culture mixture through the centrifugal separator 6 and back to the fermentor 4. The heavy phase pump 12" may be utilised for transporting separated heavy phase back to the fermentor 4. According to some embodiments, the heavy phase pump 12" may be utilised for transporting the cell culture mixture from the centrifugal separator 6 back to the fermentor 4. According to some embodiments, the heavy phase pump 12" may be utilised for transporting separated heavy phase to the receiving vessel 18. The light phase pump 12'" may be utilised for transporting separated light phase to the receiving container 8. Also, the centrifugal separator 6 as such may have a pumping effect on one or more of the cell culture mixture and the separated light phase.

- The flow control equipment 16 may comprise one or more of a first valve arrangement 14' arranged at the first conduit section 21, a second valve arrangement 14" arranged at the second conduit section 22, and a third valve arrangement 14'" arranged at the third conduit section 23. In this manner, further control over the liquids flowing in the bioprocessing system 2 may be provided. For instance, the first valve arrangement 14' may control the flow of cell culture mixture from the fermentor 4 to the centrifugal separator 6. The second valve arrangement 14" may control the flow of heavy phase and/or the cell culture mixture from the centrifugal separator 6 back to the fermentor 4 and/or to the receiving vessel 18. The third valve arrangement 14'" may control the flow of light phase from the centrifugal separator 6 to the receiving container 8 or to an optional waste liquid container 60.

In the embodiments of **Fig. 1,** each of the illustrated valve arrangements 14' - 14'" may comprise a single controllable valve, which continuously or incrementally may control the flow of cell culture mixture, separated heavy phase, or separated light phase. In the embodiments of **Fig. 2,** each of the illustrated valve arrangements 14' - 14'" may comprise a bank of valves or multiple inlet/outlet valves, which continuously or incrementally may control the flow of cell culture mixture, separated heavy phase, or separated light phase. One or more of the valve arrangements 14' - 14'" of the embodiments of **Fig. 1** may alternatively be utilised in the embodiments of **Fig. 2,** e.g. depending on the number and functions of container/s, vessel/s, and conduits included in the relevant bioprocessing system. The valve arrangements 14' - 14'" may comprise hygienic valves and/or valve banks, e.g. fulfilling sanitary requirements of the biotech industry.

Similarly, the conduits and pumps discussed herein as well as the centrifugal separator may fulfil the same sanitary requirements.

- The feed pump 12' may be a centrifugal pump, and each of the heavy phase pump 12" and the light phase pump 12'" may be a peristaltic pump. In this manner, gentle handling of the cell culture mixture may be provided by the centrifugal pump, in particular when operated at low rotational speeds. Thus, the cell culture mixture may be subjected to low shear forces not damaging the cells of the cell culture mixture. The peristaltic pumps may also function as flowmeters and thus, provide flow data for the separated light and heavy phases.

- The conduit system 10 may comprise a fourth conduit section 24 extending to the first conduit section 21. The fourth conduit section 24 may be configured for supplying a liquid other than the cell culture mixture to the inlet of the centrifugal separator 6. In this manner, the bioprocessing system 2 may be configured for supplying a different liquid than the cell culture mixture to the centrifugal separator 6, e.g. during a start-up procedure of the bioprocessing system 2, so-called priming. For instance, the liquid may be a priming liquid, such as a growth medium, to be supplied to the centrifugal separator 6 before separation of the cell culture mixture in the centrifugal separator 6 commences. Such a liquid may be stored in a separate container 30.

During such priming of the bioprocessing system, the priming liquid exiting a light phase outlet of the centrifugal separator 6 may be directed to a waste liquid container 60.

- The conduit system 10 may comprise a fifth conduit section 25 extending from the second conduit section 22 to the receiving vessel 18. In this manner, separated heavy phase may be transported from an outlet for heavy phase of the centrifugal separator 6 via the second and fifth conduit sections 22, 25 to the receiving vessel 18.

- The centrifugal separator 6 may be arranged on the fermentor 4. In this manner, a compact bioprocessing system 2 may be provided. The centrifugal separator 6 arranged on the fermentor 4 is shown in the embodiments of **Fig. 1.** Also in the embodiments of **Fig. 2,** the centrifugal separator 6 may be arranged on the fermentor 4. Conversely, the centrifugal separator 6 in the embodiments of **Fig. 2** may be arranged separate from the fermentor 4.

- The control system 16 may be configured to control the fermentor 4, such as a pump (not shown) of the fermentor 4, a valve (not shown) of the fermentor 4, an air inlet 32 to the fermentor 4, and/or a stirring member 34 arranged in the fermentor 4. In this manner, a compact, control system 16 for the bioprocessing system 2 may be provided.

- Alternatively, or additionally, the control system 16 may be configured for controlling the centrifugal separator 6 and the flow of liquids to and from the centrifugal separator 6. The fermentor 4 and the culture process therein may be controlled by a separate to control system. In such embodiments, the control system 16 of the centrifugal separator 6 and a separate control system of the fermentor 4 may communicate with each other.

**Fig. 3** illustrates embodiments of a centrifugal separator 6 to be utilised in the bioprocessing system 2 discussed herein, inter alia with reference to **Figs. 1 and 2.**

The centrifugal separator 6 is a modular centrifugal separator 6. The modular centrifugal separator 6 comprises a base unit 40 and an exchangeable separation insert 42. In **Fig. 3** a first exchangeable separation insert 42' is mounted in the base unit 40 and a second exchangeable separation insert 42" is shown beside the centrifugal separator 6.

Thus, the centrifugal separator 6 is configured for single use. That is, after use, the first exchangeable separation insert 42' in the base unit 40 is exchanged with the second exchangeable separation insert 42". The first exchangeable separation insert 42' may thereafter be disposed of. For instance, the first exchangeable separation insert 42' may be exchanged after one culture period.

During use of the modular centrifugal separator 6, the liquid feed mixture, the heavy phase, and the light phase only come into contact with the relevant first or second exchangeable separation insert 42', 42". Naturally, conduits in the form of tubes 10 configured for conducting the liquid feed mixture to the exchangeable separation insert 42', 42 " and for conducting the heavy phase and the light phase from the exchangeable separation insert 6 also come into contact with the liquid feed mixture and the heavy and light phases. The base unit 4 does not come into contact with the liquid feed mixture or any of the heavy and light phases.

Tubes 44 connected to the exchangeable separation insert 42', 42" may form part of the exchangeable separation insert 42', 42". The tubes 44 form part of the conduit system 10, see **Figs. 1 and 2,** and thus, lead to an inlet for cell culture mixture of the centrifugal separator 6 and lead from respective outlets for separated light and heavy phases of the centrifugal separator 6.

The base unit 40 comprises components for supporting and rotating the exchangeable separation insert 42', 42". Thus, the base unit 40 comprises inter alia a stationary frame 46 a rotatable member 47, and a drive unit for rotating the rotatable member (not shown).

The stationary frame 46 is stationary during use of the modular centrifugal separator. However, the base unit 40 as such may be movable, e.g. in order to be positioned at different locations at a production facility of a user. For this purpose, the stationary frame 46 may be provided with wheels 48.

The exchangeable separation insert 42', 42" is configured for part of it, such as a rotor casing 43, to be arranged inside an inner space of the rotatable member 47 of the base unit 46. Together, the rotor casing 43 of the exchangeable separation insert 42', 42" and the rotatable member 47 of the base unit 46 form a rotor 49 of the centrifugal separator 6. The exchangeable separation insert 42', 42" may also comprise one or more stationary portions 45, 45', such as portions where the tubes 44 connected to the exchangeable separation insert 42', 42".

The tubes 44 may be arranged differently than in the illustrate embodiments. Any configuration of one, two, or three tubes 44 may be provided at a stationary part of the exchangeable separation insert 42', 42". In the latter case, with three tubes at one stationary part, the exchangeable separation insert may comprise only one stationary part, at its upper or lower end.

The rotor 49 of the centrifugal separator 6 is configured to rotate about a rotational axis 51. The rotor casing 43 of the exchangeable separation insert 42', 42" delimits a separation space therein. The exchangeable separation insert 42', 42" comprises a surface enlarging insert, such as e.g., a stack of frustoconical separation discs 53 arranged in the separation space. Thus, the centrifugal separator 6 is a disc stack centrifugal separator 6.

The separation space forms part of the interior of the centrifugal separator 6, into which and from which liquids are conducted.

In a known manner, during separation operation of the centrifugal separator 6, the rotor 49 is brought into rotation around the rotational axis. The liquid feed mixture to be separated, in this case the cell culture mixture to be separated is supplied via an inlet of the centrifugal separator 6 into an interior of the centrifugal separator 6 and the separation space. Due to a density difference between the cells and the liquid in which they are suspended, the cell culture mixture is separated into a liquid light phase and a liquid heavy phase. This separation is facilitated by the interspaces between the separation discs of the stack arranged in the separation space. The separated light phase may be substantially free of cells of the cell culture mixture and may contain debris small in size than the cells of the cell culture mixture. The separated heavy phase comprises particles, such as the cells of the cell culture mixture and some debris smaller than cells. The heavy phase forms a cell containing suspension comprising a concentrated mixture of light phase and cells.

For a more detailed description of an example modular centrifugal separator to be utilised as the present centrifugal separator 6, reference is made to EP 3666394, the disclosure of which is incorporated herein by reference.

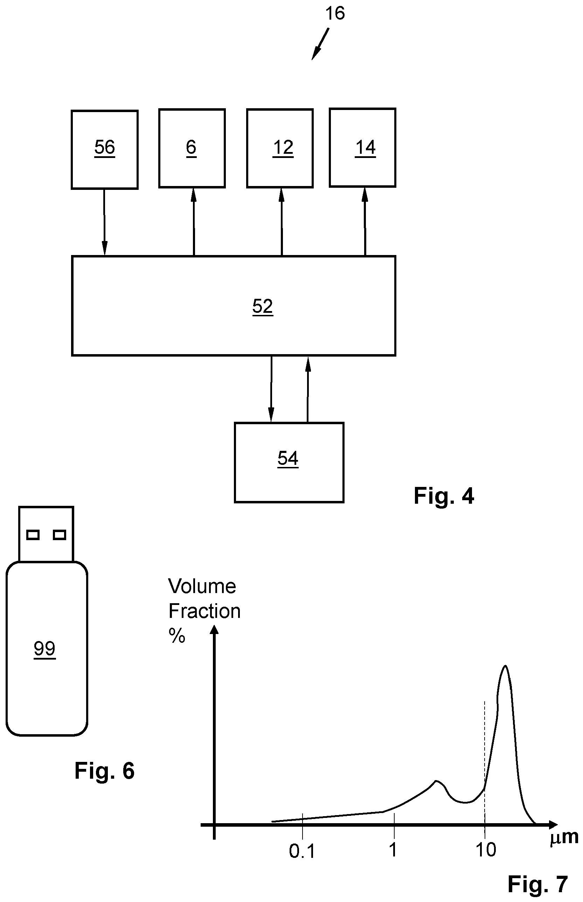

**Fig. 4** illustrates a control system 16 according to embodiments to be utilised in connection with the different aspects and/or embodiments of the invention. The control system 16 is also indicated in **Figs. 1 and 2.** The control system 16 comprises at least one control unit 52, which may take the form of substantially any suitable type of processor circuit or microcomputer, e.g. a circuit for digital signal processing (digital signal processor, DSP), a Central Processing Unit (CPU), a processing unit, a processing circuit, a processor, an Application Specific Integrated Circuit (ASIC), a microprocessor, or other processing logic that may interpret and execute instructions.

The control system 16 comprises a memory unit 54. The control unit 52 is connected to the memory unit 54, which provides the control unit 52 with, e.g. stored programme code, data tables, and/or other stored data which the control unit 52 requires to enable it to do calculations and to control the centrifugal separator 6 and the centrifugal separator the control equipment 12, 14. The control unit 52 is also adapted to store partial or final results of calculations in the memory unit 54. The memory unit 54 may comprise a physical device utilised to store data or programs, i.e. sequences of instructions on a temporary or permanent basis. According to some embodiments, the memory unit 54 may comprise integrated circuits comprising silicon-based transistors. The memory unit 54 may comprise e.g. a memory card, a flash memory, a USB memory, a hard disc, or another similar volatile or non-volatile storage unit for storing data such as e.g. ROM (Read-Only Memory), PROM (Programmable Read-Only Memory), EPROM (Erasable PROM), EEPROM (Electrically Erasable PROM), etc. in different embodiments.

The control system 16 may further comprise one or more sensors 56 (only one shown in Fig. 4). The control unit 52 receives measured data, such as e.g. centrifugal separator rotational speed from the one or more sensors 56. The control system 16 sends control data to the centrifugal separator 6 and the control equipment 12, 14 of controlling e.g. centrifugal separator rotational speed, pump speeds, and valve arrangement settings.

Thus, the control unit 52 is configured to receiving input signals from the sensor/s 56 and to send output signals to the centrifugal separator 6 and the control equipment 12, 14. These signals may comprise waveforms, pulses or other attributes, which can be detect and sent as information by the control unit 52, and which can be directly or indirectly converted to signals processable by the control unit 52. Each of connections to the sensor/s, the centrifugal separator 6, and the control equipment 12, 14 may take the form of one or more from among a cable, a data bus, e.g. a CAN (controller area network) bus, a MOST (media orientated systems transport) bus or some other bus configuration, or a wireless connection. In the embodiment depicted, only one control unit 52 and memory 54 are shown, but the control system 16 may alternatively comprise more than one control unit and/or memory.

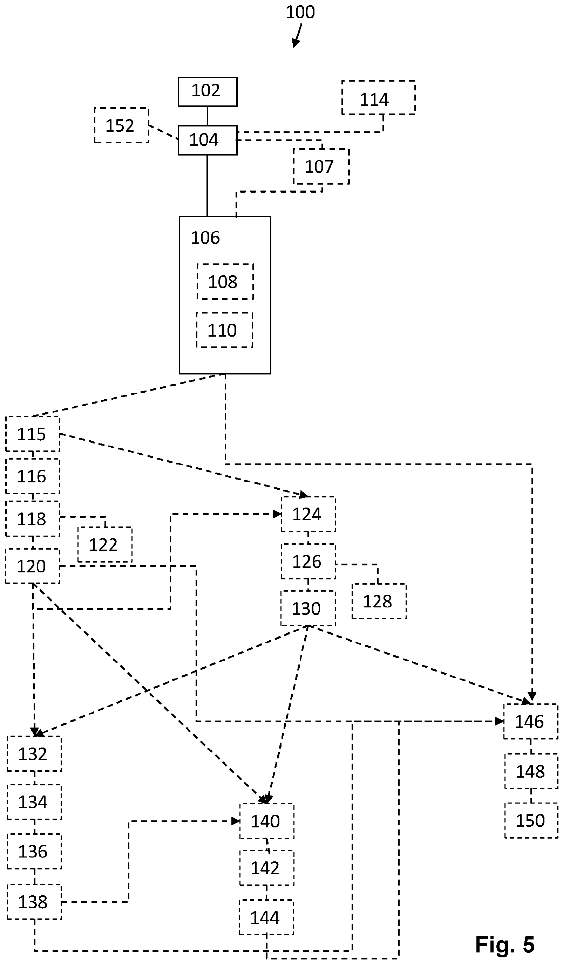

In the following, the control system 16 and its operation is discussed, inter alia with reference to **Figs. 1** - **4.** Moreover, the control system 16 may be configured to perform embodiments of a method 100 as discussed herein, inter alia with reference to **Fig. 5** Hence, advantages discussed in connection with the method 100 may be applicable in the control system 16 and vice versa.

The control system 16 is configured to:

- conduct a flow of cell culture mixture from the fermentor 4 into an interior of the centrifugal separator 6, and simultaneously,
- continuously return from the interior of the centrifugal separator 6 a flow of liquid to the fermentor 4. In this manner, perfusion mode operation of the bioprocessing system 2 comprising the centrifugal separator 6 comprising a surface enlarging insert is enabled. More specifically, in this manner stagnant liquid in the centrifugal separator 6 is avoided, which otherwise when returned to the fermentor 4 would degrade the cell culture mixture in the fermentor 4 during the culture period.

The liquid may be heavy phase separated from the cell culture mixture. Thus, the heavy phase, containing a more concentrated cell culture mixture than the cell culture mixture led into the interior of the centrifugal separator 6, may be returned to the fermentor 4. According to some embodiments, intermittently, the liquid may be the cell culture mixture as led into the interior of the centrifugal separator 6, i.e. flowing through the centrifugal separator 6 without being separated therein.

The flow of cell culture mixture may be conducted from the fermentor 4 to the centrifugal separator 6 via the first conduit section 21 and via the feed pump 12' and/or the first valve arrangement 14', if present in the system 2.

The flow of liquid may be conducted/returned from the centrifugal separator 6 via the second conduit section 22 and the heavy phase pump 12", and optionally, via the second valve arrangement 14", if present in the system 2.

According to embodiments, the control system 16 may be configured during a first time period to:

- run the centrifugal separator 6 to separate the flow of cell culture mixture into a light phase and a heavy phase, and
- continuously return from the interior of the centrifugal separator 6 the heavy phase separated in the centrifugal separator 6 as the flow of liquid to the fermentor 4. In this manner, perfusion mode operation of the bioprocessing system 2 may be provided by returning the cells of the cell culture mixture with the heavy phase back to the fermentor 4. In the fermentor 4, the returned cells may continue producing the sought-after substance of the relevant bioprocess.

According to embodiments, the control system 16 may be configured during the first time period to:

- conduct the light phase separated in the centrifugal separator 6 to the receiving container 8. In this manner, the light phase may be collected in the receiving container 8, e.g. for further downstream processing of the light phase or expecting therefrom the sought-after substance of the bioprocess.

The light phase that has been conducted to the receiving container 8 may be replaced with fresh growth medium, e.g. to the fermentor 4 via a growth medium conduit 33. Mentioned as an example, a turnover of the volume of the fermentor 4 may be one to two times per 24 hours. That is, the light phase separated from the cell culture mixture that is conducted to the receiving container 8 may be equal to the volume of the fermenter 4 or twice the volume of the fermenter 4 per 24 hours.

Once filled, the receiving container 8 may be replaced with an empty receiving container. This may be done one or more times over the culture period. The contents of the filled receiving container 8, i.e. the light phase therein may be subjected to downstream processing. An alternative of, or a complement to, replacing the receiving container 18 may be to intermittently empty the receiving container 8 and transfer the light phase for downstream processing.

According to embodiments, the control system 16 may be configured during a second time period to:

- continuously return from the interior of the centrifugal separator 6 the cell culture mixture unseparated, as the flow of liquid to the fermentor 4. In this manner, a continuous flow of unseparated cell culture mixture may be maintained through the centrifugal separator 6. More specifically, during the second time period no separation of the cell culture mixture takes place in the centrifugal separator 6. Thus, stagnant liquid in the centrifugal separator 6 may be prevented during a time period when no separation of the cell culture mixture is required in the bioprocessing system 2. Accordingly, conditions for perfusion mode operation of the bioprocessing system 2 may be achieved also e.g. when the bioprocess in the system 2 does not provide for continuous extraction of light phase / product. This may be due to a high separation capacity of the centrifugal separator 6 in comparison with the production rate in the fermentor 4 of cell culture mixture ready for extraction of sought-after substance.

According to embodiments, the control system 16 may be configured during the second time period, to maintain a rotor of the centrifugal separator 6 standing still, or rotate the rotor at a lower rotational speed than during the first time period, or rotate the rotor in a rotational direction opposite to a rotational direction during the first time period. In this manner, no separation of the cell culture mixture may take place in the rotor of the centrifugal separator 6 during the second time period.

If the rotor rotates during the second time period, its rotational speed is substantially lower than the rotational speed during the first time period. This applies the respectively of the rotational direction of the rotor. For instance, the rotational speed during the second time period may be within a range of 1 - 10%, or within a range of 0.1 - 20% of the rotational speed during the first time period. Maintaining such rotational of the rotor may ensure that no region with stagnant cell culture mixture is formed in the separation space during the second time period.

According to embodiments, the control system 16 may be configured to alternate the first and second time periods. In this manner, perfusion mode operation of the bioprocessing system 2 may be maintained over longer periods of time, during which light phase/product is intermittently extracted from the cell culture mixture in the fermentor 4. That is, for each first time period, light phase/product is separated in the centrifugal separator 6 and for each second time period, extraction of light phase/product is set on hold while unseparated cell culture mixture is returned to the fermentor from the centrifugal separator 6.

According to embodiments, wherein the bioprocessing system 2 comprises a receiving vessel 18, the control system 16 may be configured during a third time period to:

- stop the continuous return from the interior of the centrifugal separator 6 of the flow of liquid to the fermentor 4,
- conduct a flow of the cell culture mixture from the fermentor 4 to the centrifugal separator 6,
- run the centrifugal separator 6 to separate the flow of cell culture mixture in the centrifugal separator 6 into a light phase and a heavy phase, and
- conduct the heavy phase separated in the centrifugal separator 6 to the receiving vessel 18. In this manner, the concentration of cells in the cell culture mixture in the fermentor 4 may be reduced. More specifically, over the culture period, the cell content of the cell culture mixture may increase and may reach levels, which have a negative effect on the cell culture mixture and/or production of the sought-after substance. In such situations, the cell concentration of the cell culture mixture in the fermentor 4 may be reduced by diverting the separated heavy phase and the cells contained therein from the fermentor 4 during the third time period.

In the comparison with the volume of the separated light phase conducted to the receiving container 8, the separated heavy phase conducted to the receiving vessel 18 may be small. Thus, the separated heavy phase conducted to the receiving vessel 18 may only contribute to a minor portion of the turnover of the volume of the fermenter 4.

The third time period may be followed by a second time period or a first time period and the measures performed in connection with said time periods. The third time period and the measures performed in connection therewith may be repeated intermittently over the culture period in order to maintain the cell concentration of the cell culture mixture within a desired range.

According to embodiments, the control system 16 may be configured during the third time period to:

- conduct the light phase separated in the centrifugal separator 6 to the receiving container 8. In this manner, also during the third time period, when separated heavy phase may be diverted to the receiving vessel 18, the separated light phase may be collected in the receiving container 8. Namely, also during the third time period the light phase / product may contain the sought-after substance.

According to embodiments, the centrifugal separator 6 comprises a rotor (as mentioned above) and the control system 16 may be configured to rotate the rotor at a higher rotational speed during the third time period than during the first time period. In this manner, a more concentrated heavy phase may be separated during the third time period than during the first time period. Accordingly, less light phase may be contained in the suspension forming the separated heavy phase during the third time period than during the first time period, and thus, less light phase may be sent to the receiving vessel 18 and potentially be wasted from the bioprocess. Consequently, more light phase may be separated during the third time period than during the first time period.

According to embodiments, the control system 16 may be configured during a fourth time period to:

- stop the continuous return from the interior of the centrifugal separator 6 of the flow of liquid to the fermentor 4, if not done previously,
- conduct all of the cell culture mixture from the fermentor 4 to the centrifugal separator 6,
- run the centrifugal separator 6 to separate the cell culture mixture into a light phase and a heavy phase, and
- conduct the light phase separated in the centrifugal separator 6 to the receiving container 8. In this manner, the fermentor 4 may be emptied at an end of the relevant culture period and a final amount of light phase / product may be extracted from the cell culture mixture in the fermentor 4.

According to embodiments, the control system 16 may be configured during the fourth time period to:

- conduct the heavy phase separated in the centrifugal separator 6 to the receiving vessel 18. In this manner, the heavy phase separated during the fourth time period may be collected in the receiving vessel 18 and not sent back to the fermentor 4. Thus, the fermentor 4 is emptied during the fourth time period.

According to embodiments, the bioprocessing system 2 may optionally comprise a centrifugal apparatus 6' and optionally the conduit system 10 may interconnect the receiving container 8 with the centrifugal apparatus 6'. The control system 16 may be configured subsequent to any one of the first to sixth time period to:

- conduct the light phase collected in the receiving container 8 to the centrifugal separator 6 or the centrifugal apparatus 6',
- run the centrifugal separator 6 or the centrifugal apparatus 6' to separate the light phase into a light fraction and a heavy fraction, and
- transfer the light fraction from the centrifugal separator 6 or the centrifugal apparatus 6'. In this manner, the light phase collected in the receiving container 8 may be cleaned from debris that it may contain and thus, prepare the light phase for downstream processing by presenting it in the form of the separated light fraction.

The transfer of the light fraction may be to a further receiving container 50 or directly to downstream processing equipment for further processing of the light fraction, such as extracting a sought-after substance from the light fraction.

The centrifugal apparatus 6' is schematically indicated in **Fig. 2** and forms an optional different centrifugal apparatus 6' other than the centrifugal separator 6 utilised for separating the light and heavy phases during the first time period. Accordingly, either the same centrifugal separator 6 or a different centrifugal apparatus 6' may be utilised for separating the light and heavy fractions. Such a different centrifugal apparatus 6' may also be a centrifugal separator, of the same or a different kind as the centrifugal separator 6. The centrifugal apparatus 6' may be configured for single use.

The conduit system 10 may comprise a sixth conduit section 26 for conducting the light phase from the receiving container 8 to the centrifugal separator 6 or the centrifugal apparatus 6'.

If the same centrifugal separator 6 is utilised for separating the light and heavy fractions as during the first time period, the conduit system 10 may comprise a seventh conduit section 27 connected to the third conduit section 23 and the further receiving container 50. Thus, the separated light fraction may be transferred via the seventh conduit section 27 to the further receiving container 50. In such embodiments, the separated heavy fraction may be conducted to the receiving vessel 18 or a different waste receptacle connected to the heavy phase outlet of the centrifugal separator 6.

In order to ensure a low particle content of the separated light fraction, the centrifugal apparatus 6' may have a higher separation capacity then the centrifugal separator 6.

An advantage of utilising the centrifugal apparatus 6' may be that it may be operated in parallel with the centrifugal separator 6. Accordingly, the receiving container 8 may be emptied or relieved of some of its contents during the culture period.

According to embodiments, the control system 16 may be configured during the first and second time periods to provide a flow of the cell culture mixture at substantially a same flowrate, $q$, into the interior of the centrifugal separator 6. In this manner, there may be substantially no difference in flowrate, $q$, between the first and second time periods, which may promote gentle handling of the cell culture mixture and the separated heavy phase during each of the first and second time periods. Specifically, the flowrate, q, may be selected for providing low shear stress on the cell culture mixture and the separated heavy phase in the conduit system 10.

The flowrate, q, may differ between the first and second time periods within a range of +/-10%.

As discussed above, according to embodiments, the centrifugal separator 6 comprising a surface enlarging insert may be a modular centrifugal separator comprising a base unit 40 and an exchangeable separation insert 42', 42" mounted in the base unit 40.

According to embodiments, wherein the conduit system 10 comprises the fourth conduit section 24 being configured for supplying a liquid other than the cell culture mixture to the inlet of the centrifugal separator 6, the control system 16 may be configured prior to the first time period to:

- run the feed pump 12', the heavy phase pump 12", and the light phase pump 12"',
- provide a liquid passage from the fourth conduit section 24 to the first conduit section 21 and the centrifugal separator 6 utilising the first valve arrangement 14',
- provide a diverting passage from the second conduit section 22 utilising the second valve arrangement 14", and
- provide a diverting passage from the third conduit 23 utilising the third valve arrangement 14"',

such that the liquid other than cell culture mixture is transferred via the fourth and first conduit sections 24, 21 to the interior of the centrifugal separator 6 and out of centrifugal separator 6 via the heavy phase outlet and the light phase outlet and being diverted from the second and third conduit sections 22, 23 before reaching the fermentor 4 and the receiving container 8. In this manner, the control system may be configured to supply a different liquid than the cell culture mixture to the centrifugal separator 6 prior to the first time period. Thus, a priming liquid, such as a growth medium, may be supplied to the centrifugal separator 6 before separation of the cell culture mixture in the centrifugal separator 6 and priming of the centrifugal separator 6 may be done with a less costly/valuable liquid than the cell culture mixture. The priming liquid may be provided in a separate container 30 and conducted via the fourth conduit section 24 to the centrifugal separator 6.

Diverting of the liquid other than cell culture mixture from the light phase outlet may be to the waste liquid container 60. Diverting of the liquid other than cell culture mixture from the heavy phase outlet may be to the receiving vessel 18.

According to embodiments, the control system 16 during the first time period may be configured to:

- run the feed pump 12', the heavy phase pump 12", and the light phase pump 12"',
- provide a liquid passage from the fermentor 4 to the first conduit section 21 and the centrifugal separator 6 utilising the first valve arrangement 14',
- provide a heavy phase passage via the second conduit section 22 to the fermentor 4 utilising the second valve arrangement 14", and

provide a light phase passage via the third conduit 23 to the receiving container 8 utilising the third valve arrangement 14"'. In this manner, the control system 16 may be configured to transfer the cell culture mixture from the fermentor 4 to the centrifugal separator 6. The therein separated light and heavy phases may in this manner also be transferred to the receiving container 8 and fermentor 4, respectively.

According to embodiments, the control system 16 may be configured during the second time period to:

- run the feed pump 12' and the heavy phase pump 12",
- maintain the first and second valves arrangements 14', 14" open, and
- maintain the third valve arrangement 14"' closed, such as to provide a cell culture mixture passage from the fermentor 4 via the first conduit section 21 to and through the centrifugal separator 6 and therefrom via the second conduit section 22 returned back to the fermentor 4. In this manner, the control system 16 may be configured for preventing stagnant cell culture mixture in the centrifugal separator 6 when no separation takes place therein. Thus, cell culture mixture may not degrade during the second time period when no separation of the cell culture mixture takes place in the centrifugal separator 6.

According to embodiments, wherein the conduit system 10 comprises the fifth conduit section 25 and the receiving vessel 18, the control system 16 may be configured during the third time period to:

- run the feed pump 12' and the heavy phase pump 12",
- run the centrifugal separator 6, and
- provide a heavy phase passage from the heavy phase outlet to the receiving vessel 18 utilising the second valve arrangement 14" and the fifth conduit section 25, such that the cell culture mixture is transferred from the fermentor

4 via the first conduit section 21 to the centrifugal separator 6, separated in the centrifugal separator 6 into a light phase which is conducted via the third conduit section 23 to the receiving container 8 and a heavy phase which is conducted via the first and fifth conduit 21, 25 sections to the receiving vessel 18. In this manner, the control system 16 may be configured for diverting separated heavy phase from the fermentor 4. Instead of being transferred to the fermentor 4, the separated heavy phase may be transferred from the centrifugal separator 6 via the second and fifth conduit sections 22, 25 to the receiving vessel 18.

**Fig. 5** illustrates embodiments of a method 100 for operating a bioprocessing system. The bioprocessing system may be a system 2 according to any one of embodiments discussed in connection with **Figs. 1 - 3,** and comprises a control system 16 as discussed in connection with **Figs. 1 - 4.** In the following reference is also made to **Figs. 1 - 4.** Features and advantages discussed in connection with the method 100 may be applicable in the control system 16 and vice versa.

The method 100 for operating a bioprocessing system, comprises steps of:

- producing 102 a cell culture mixture in the fermentor 4,
- conducting 104 a flow of cell culture mixture from the fermentor 4 into an interior of the centrifugal separator 6 comprising a surface enlarging insert, simultaneously with the step of producing 102 the cell culture mixture, and
- returning 106 continuously from the interior of the centrifugal separator 6 a flow of liquid to the fermentor 4, simultaneously with the steps of producing 102 the cell culture mixture and conducting 104 the flow of cell culture mixture.

Thus, perfusion mode operation of the bioprocessing system 2 comprising the centrifugal separator 6 comprising a surface enlarging insert is enabled by the continuous return of a flow of liquid from the interior of the centrifugal separator 6 to the fermentor 4.

The step of producing 102 the cell culture mixture comprises cultivation of the relevant cells in the fermentor 4 in a known manner, utilising inter alia growth medium, oxygen, and favourable temperature conditions.

Accordingly, during the step of producing 102 there may be provided, continuously or intermittently one or more of:

- supplying growth medium via the growth medium conduit 33 into the fermentor 4,
- supplying oxygen via an oxygen or air inlet 32 into the fermentor 4,
- stirring the cell culture mixture in the fermenter 4 with the stirring member 34,
- controlling a temperature within the fermenter 4 utilising a cooling and/or heating element 35.

The step of returning 106 continuously from the interior of the centrifugal separator 6 the flow of liquid to the fermentor 4, may take place over more than 90%, such as more than 95 %, of a duration of the entire culture period.

According to embodiments, the method 100 may comprise during a first time period a step of:

- separating 107 the flow of cell culture mixture into a light phase and a heavy phase in the centrifugal separator, and wherein the step of returning 106 continuously from the interior of the centrifugal separator 6 a flow of liquid to the fermentor 4 may comprise during the first time period:
- returning 108 the heavy phase separated in the centrifugal separator 6 to the fermentor 4 as the flow of liquid to the fermentor 4.

Thus, during the first time period, the cell culture mixture from the fermentor 4 may be separated in into the light and heavy phases and the heavy phase containing the cells of the cell culture mixture is returned to the fermentor 4

According to embodiments, in the step of separating 107 the flow of cell culture mixture into a light phase and a heavy phase, a flow of the separated light phase may form a maximum of 5 %V/V (volume %) of the flow of cell culture mixture.

Thus, an appropriately small amount of light phase / product may be drawn from the cell culture mixture during separation of the cell culture mixture. The amount of light phase may be a function inter alia of the fermentor 4 volume, the flow of cell culture mixture from the fermentor 4, and the cell density of the cell culture mixture.

According to embodiments, the method may comprise during the first time period a step of:

- conducting 112 the light phase separated in the centrifugal separator 6 to a receiving container 8.

Thus, the light phase / product may be collected for subsequent processing thereof.

According to embodiments, during the first time period, the heavy phase may comprise a growth medium and whole cells and the light phase may comprise the growth medium and debris from the cell culture mixture of a particle size smaller than whole cells and may be substantially free of whole cells.

The growth medium may be in various stages of being consumed by the cells. However, during the culture period, fresh growth medium is supplied to fermentor 4 in order to promote growth of the cells. Similarly, e.g. during the first time period, the light phase comprising growth medium is extracted from the fermentor 4 to the receiving container 8. As mentioned, the light phase comprises the sought-after substance being the desired end produce once the light phase has been subject to processing downstream of the bioprocessing system 2. Accordingly, fresh growth medium also replaces light phase that has been conducted to the receiving container 8

According to embodiments, during the first time period, the flow of the cell culture mixture may be conducted at a flowrate, q, wherein the centrifugal separator 6 may be operated at an Area Equivalent, Ae, such that at the flowrate, q, whole cells having a diameter d are separated from the cell culture mixture to the heavy phase. In this manner, the cells in the cell culture mixture may be separated with the heavy phase as they pass through the centrifugal separator 6 and may be returned to the fermentor 4. Smaller particles, such as debris e.g., debris from cell lysis, may follow with the light phase as well as the heavy phase.

Accordingly, the critical particle, d, during the first time period may be selected smaller than the diameter, d, of the whole cells of the cell culture mixture. Mentioned as an example, the diameter, d, of the whole cells may be within a range of 10 - 30 $\mu$m.

More specifically, the area equivalent, Ae, is a measure of the separation capacity of the centrifugal separator 6 comprising a surface enlarging insert. The higher the area equivalent, the higher the separation performance of the centrifugal separator 6.

The area equivalent, Ae, here exemplified in the context of a centrifugal separator comprising a stack of separation discs, is calculated according to the following formula:

$$\mathbf{Ae} = \frac{2\pi N\omega^2\left(r_2^3 - r_1^3\right)}{3g\tan\alpha} \qquad (1)$$

Wherein: g = gravity constant, N = the number of frustoconical separation discs of the separation insert, $\alpha$ = half the apex angle of the frustoconical separation discs, i.e. the angle between the rotational axis and an inner surface of one of the frustoconical separation discs, $\omega$ = rotational speed of the rotor of the centrifugal separator 6, $r_2$ = outer radius of the frustoconical separation disc, $r_1$ = inner radius of the frustoconical separation disc.

Since the Ae has a rotational speed component, $\omega$, the Ae may be manipulated by adjusting the rotational speed of the rotor of the centrifugal separator 6.

The critical particle according to Ambler in 1959 and later Axelsson/Madsen describes the diameter, d, of the largest particle in the suspension, i.e. in this case in the cell culture mixture, which is separated with the light phase.

The following equations and relationships are valid:
The flowrate, q, of suspension through the centrifugal separator is given by:

$$q = v_g \, Ae \qquad (2)$$

where $v_g$ is the Stokes settling velocity and Ae is the area equivalent as given by equation (1) above.

Stokes law provides:

$$\mathbf{V_g} = \frac{\Delta\rho d^2 g}{18\mu} \, [m/s] \qquad (3)$$

where $\rho$ is the density difference of the relevant particle and its surrounding medium, d is the particle diameter, and $\mu$ is the viscosity of the suspension.

The critical particle, d, may be calculated from equations (1), (2) and (3).

Accordingly, the critical particle, d, is a function of the rotational speed, $\omega$, of the rotor of the centrifugal separator 6 as well as the flowrate, q.

**Fig. 7** is a diagram illustrating an example of the particle size distribution of the cell culture mixture in the fermenter 4. In the example, the size of the whole cells is approximately 11 $\mu$m, as indicated by the large peak of the curve. The smaller peak of the curve indicates the main size of the debris contained in the cell culture mixture. However, debris within a range of <0.1 - 10 $\mu$m is contained in the cell culture mixture, such as debris from cell lysis. In the example, a suitable size of the critical particle, d, would be 10 $\mu$m, as indicated by the broken line.

Thus, the whole cells are separated with the heavy phase. The debris of smaller size is contained in both the heavy and light phases. Accordingly, part of the debris is removed from the fermentor 4 with the separated light phase and thus, from the fermentation process therein.

In the cell culture mixture, like in any Newtonian fluid, viscous stresses in the form of shear stress arises from its flow. A maximum shear stress that the cell culture mixture and/or the separated heavy phase may be subjected to as it/they flow through the conduit system 10 may be that which does not damage the cells contained therein. The flowrate in the conduit system 10 may be limited depending on the shear stress that the cells may be subjected to therein. For instance, the flowrate may be limited to a flowrate at which at least 95% of the cells survive passing through the conduit system.

In the centrifugal separator 6, the cell culture mixture and the separated heavy phase are subjected to highest shear stress at the inlet for the cell culture mixture and at the heavy phase outlet, where the cell culture mixture and the separated heavy phase are accelerated and decelerated. An inlet and outlet concentrical with the rotational axis of the rotor of the centrifugal separator 6 provides the gentlest acceleration and deceleration.

Returning to **Fig. 5** and **Figs. 1 - 4**, according to embodiments, preceding the first time period, the method 100 may comprise a step of:

- priming 114 the centrifugal separator 6 with a liquid other than cell culture mixture, such as a growth medium. In this manner, the priming 114 of the centrifugal separator 6 with the liquid other than cell culture mixture may ensure an even flow of the cell culture mixture into the interior of the centrifugal separator 6 at the beginning of the first time period.

In the step of priming 114, the interior of the centrifugal separator 6, such as the separation space of the centrifugal separator 6, may be filled with the liquid other than cell culture mixture.

The step of priming 114 may also include priming parts of, or the entire, conduit system 10, such as conduit sections leading from and/or to the fermentor 4. Thus, the cell culture mixture may be gently introduced in such conduit sections leading into the interior of centrifugal separator 6 and the separated heavy phase may be gently introduced into such conduit sections leading from the centrifugal separator 6 back to the fermentor 4.

According to embodiments, the step of returning 106 continuously from the interior of the centrifugal separator 6 a flow of liquid to the fermentor 4 may comprise during a second time period a step of:

- returning 110 the flow of cell culture mixture from the interior of the centrifugal separator 6 to the fermentor 4 as the flow of liquid to the fermentor 4.

During the second time period, no or substantially no separation takes place in the centrifugal separator 6. The cell culture mixture simply flows through the centrifugal separator 6.

In this manner, there may be provided for different operation of the centrifugal separator 6 during the first and second time periods. During both time periods liquid is returned from the centrifugal separator 6 to the fermentor 4. Thus, stagnant cell culture mixture in the centrifugal separator 6 may be avoided during perfusion mode operation of the bioprocessing system 2, irrespectively of whether separation takes place in the centrifugal separator 6 or not.

During the second time period, a flow direction of the cell culture mixture within the bioprocessing system 2 may be in a first flow direction from the fermentor 4 via the first conduit section 21 to the centrifugal separator 6 and back to the fermentor 4 via the second conduit section 22. The feed pump 12' and the heavy phase pump 12" may pump the cell culture mixture in the first flow direction. Alternatively, the heavy phase pump 12" may be standing still and simply open for flow therethrough and that the feed pump 12' alone pumps the cell culture mixture in the first flow direction.

Alternatively, the flow direction during the second time period may be in a second flow direction, opposite to the first flow direction, i.e. from the fermentor 4 via the second conduit section 22 to the centrifugal separator 6 and back to the fermentor 4 via the first conduit section 21. The second flow direction may be available if the feed pump 12' and/or the heavy phase pump 12" is/are reversible.

According to embodiments, during the second time period during the step of returning 110 the flow of cell culture mixture, a rotor of the centrifugal separator may be standing still or rotating at a lower rotational speed than during the first time period during the step of separating 107 the flow of cell culture mixture, or rotating in a rotational direction opposite to a rotational direction during the first time period during the step of separating 107 the flow of cell culture mixture. In this manner, it may be ensured that no separation or substantially no separation takes place in the centrifugal separator during the second time period. Thus, build-up of separated cells may be avoided.

Naturally, in case of rotation of the rotor in the rotational direction opposite to the rotational direction during the first time period, the rotational speed is lower than the rotational speed than during the first time period in order to prevent or at least substantially reduce separation of cells in the separation space of the centrifugal separator.

If rotating during the second time period, a rotational speed of the rotor of the centrifugal separator 6 may be within a range of 0.1 - 10% of the rotational speed of the rotor during the first time period.

An advantage of rotating the rotor of the centrifugal separator 6 in the rotational direction opposite to the rotational direction during the first time period may be to loosen or release separated cells at a periphery of the separation space of the centrifugal separator 6. Thus, the cells may be transported back to the fermentor 4 with the cell culture mixture flowing through the interior of the centrifugal separator 6 during the second time period.

Similarly, an advantage of applying a reverse flow direction, i.e. applying the second flow direction discussed above, during the second time period, may be to avoid stagnant regions within the interior of the centrifugal separator 6, which depending on the construction of the centrifugal separator 6 may form when the rotor is standing still or rotating at a substantially reduced rotational speed.

According to embodiments, during the first and second time periods, the flow of the cell culture mixture may be conducted at substantially a same flowrate, q, into the interior of the centrifugal separator.

For instance, the flowrate, q, may not differ more than +/- 10% between the first and second time periods.

According to embodiments, the method 100 may comprise during a third time period steps of:

- stopping 115 the step of returning 106 continuously from the interior of the centrifugal separator a flow of liquid to the fermentor,
- conducting 116 a flow of the cell culture mixture from the fermentor 4 to the centrifugal separator 6,
- separating 118 the flow of cell culture mixture in the centrifugal separator 6 into a light phase and a heavy phase, and
- conducting 120 the heavy phase separated in the step of separating 118 to a receiving vessel 18. In this manner, the cell concentration of the cell culture mixture may be reduced by directing of the heavy phase separated in the step of separating 118 to the receiving vessel 18 instead of back to the fermentor 4.

According to embodiments, the method 100 may comprise during the third time period a step of:

- conducting 122 the light phase separated in the step of separating 118 to a receiving container. In this manner, the separated light phase during the third time period may be collected for downstream processing.

The receiving container may be the receiving container 8 as discussed above and shown in **Fig. 2** or the receiving container may be a different receiving container for the light phase separated during the step of separating 118.

According to embodiments, during the third time period, a rotor of the centrifugal separator may be rotating at a higher rotational speed than during the first time period. In this manner, the reduction of cell concentration and of smaller particle content of the cell culture mixture may be efficiently performed and more light phase may be separated in the step of separating 118 during the third time period than in the step of separating 107 during the first time period.

According to embodiments, the method 100 may comprise during a fourth time period steps of:

- stopping 115 the step of returning 106 continuously from the interior of the centrifugal separator a flow of liquid to the fermentor, if not done previously,
- conducting 124 all of the cell culture mixture from the fermentor 4 to the centrifugal separator 6,
- separating 126 the cell culture mixture in the centrifugal separator 6 into a light phase and a heavy phase, the light phase forming 50 - 95 %V/V of the cell culture mixture, and
- conducting 130 the light phase separated in the centrifugal separator to a receiving container. In this manner, the fermentor may be emptied at the end of the culture period and the light phase / product may be extracted or harvested from the cell culture mixture during the fourth time period.

The receiving container may be the receiving container 8 as discussed above and shown in **Fig. 2** or the receiving container may be a different receiving container for the light phase separated during the step of separating 126.

According to embodiments, the method 100 may comprise during the fourth time period a step of:

- conducting 128 the heavy phase separated in the centrifugal separator 6 to a receiving vessel. In this manner, the heavy phase separated during the step of separating 126 may be diverted to a receiving vessel instead of being returned to the fermentor 4. Thus, the fermentor 4 may be emptied during the fourth time period.

The receiving vessel may be the receiving vessel 18 as discussed above and shown in **Fig. 2** or the receiving vessel may be a different receiving vessel for the heavy phase separated during the step of separating 126.

According to embodiments, subsequently to the third and/or fourth time period, the method 100 may comprise steps of:

- diluting 132 the heavy phase in the receiving vessel 18 with a liquid to produce a diluted heavy phase,
- transferring 134 during a fifth time period the diluted heavy phase from the receiving vessel 18 to a centrifugal apparatus, such as the centrifugal separator 6,

- separating 136 during the fifth time period the diluted heavy phase in the centrifugal apparatus into a light phase and a further heavy phase, and
- conducting 138 the light phase to a receiving container. In this manner, the heavy phase separated during one or more of the first, third, and fourth time periods may be diluted with a liquid, such as e.g. growth medium. By separating the diluted heavy phase, further light phase containing the sought-after substance may be produced to increase the yield from the bioprocess.

The receiving container may be the receiving container 8 as discussed above and shown in **Fig. 2** or the receiving container may be a different receiving container for the light phase separated during the step of separating 136.

According to embodiments, during the fifth period, the rotor of the centrifugal separator 6 may be rotated at a higher rotational speed than during the first time period. In this manner, the separated heavy phase during the fifth time period may have a higher concentration of particles than the separated heavy phase during the first time period. Accordingly, a large portion of the available light phase may be drawn from the diluted heavy phase during the fifth time period.

According to embodiments, subsequently to the third, fourth, and/or fifth time period, the method 100 may comprise during a sixth time period steps of:

- flushing 140 the centrifugal separator 6 with a liquid, such as a growth medium,
- running 142 the centrifugal separator 6, and
- conducting 144 at least part of the liquid via a light phase outlet of the centrifugal separator 6 to the receiving container 8. In this manner, remaining product containing the sought-after substance may be flushed out of the centrifugal separator and thus, may be available for utilisation.

Since the centrifugal separator 6 is running, remaining particles such as cells remain within the centrifugal separator 6 and the part of the liquid being conducted via the light phase outlet may be free of, or at least substantially free of, particles.

According to embodiments, during the step of running 142 the centrifugal separator 6, the rotor of the centrifugal separator 6 may be rotated at a higher rotational speed than during the first time period. In this manner, during the sixth time period it may be ensured that the liquid being conducted via the light phase outlet contains a minimal concentration of particles.

According to embodiments, subsequently to any one of the first to the sixth time period, the method 100 may comprise steps of:

- conducting 146 the light phase collected in the receiving container 8 to the centrifugal separator 6 or to a centrifugal apparatus 6',
- separating 148 the light phase in the centrifugal separator 6 or the centrifugal apparatus 6' into a light fraction and a heavy fraction, the heavy fraction comprising remaining debris from the cell culture mixture, and
- transferring 150 the light fraction for further processing, such as extraction of an extracellular substance from the light fraction. In this manner, the light phase collected in the receiving container 8 may be cleaned from at least most of the debris that it may contain and thus, prepare the light phase for downstream processing by presenting it in the form of the separated light fraction.

Reference is further made to the discussion above related to the corresponding feature of the control system 16.

According to embodiments, during the step of separating 148 the light phase in the centrifugal apparatus 6 into a light fraction and a heavy fraction, the rotor of the centrifugal separator 6 may be rotated at a higher rotational speed than during one or more of the first to sixth time periods. In this manner, it may be ensured that the light fraction may contain a minimal concentration of particles, i.e. debris of substantially smaller size than the diameter of the whole cells is separated from the light fraction the sixth time period.

According to embodiments, the centrifugal separator comprising a surface enlarging insert may be a modular centrifugal separator comprising a base unit 40 and an exchangeable separation insert 42', 42" mounted in the base unit 40. The method 100 may comprise a step of:

- exchanging 152 a used exchangeable separation insert 42' for an unused exchangeable separation insert 42" prior to the step of conducting 104 a flow of cell culture mixture from the fermentor 4 into the interior of the centrifugal separator 6 comprising the surface enlarging insert.

Thus, the centrifugal separator 6 may be ready for performing its separation duty or duties in the method 100 for operating the bioprocessing system 2.

As mentioned above the first and second time periods may be alternated.

Also, or alternatively, the first and third time periods may be alternated.

A further alternative may be to alternate the first, second, and third time periods.

Various embodiments of the method 100 with respect to the first through sixth time periods and thereto related steps are envisioned, depending on the requirements of the bioprocess, the relevant centrifugal separator 6 capacity, the yield of the bioprocess, the cost of operating the bioprocess, etc.

One or more of the following may apply: A second time period may follow upon a first time period. A first time period may follow upon a second time period. A third time period may follow upon a second or a first time period. A first time period may follow upon a third time period. The fourth time period may follow upon one of a first, second, or third time period. A fifth time period may follow upon one of a third or fourth time period. The sixths time period may follow one of a third, fourth, or fifth time period.

Further, the second time period and related method steps may be excluded. Accordingly, the third or the fourth time period may follow directly upon the first time period. Alternatively, the third time period and related method steps may be excluded. Accordingly, the fourth time period may follow directly upon the first or second time period. Further, each of the fifth and sixth time periods and thereto related steps is optional.

According to a further aspect, there is provided a method for operating a bioprocessing system, the bioprocessing system comprising a fermentor for producing therein a liquid cell culture mixture using a growth medium, and a centrifugal separator comprising a surface enlarging insert, wherein the method comprising steps of:

- conducting continuously during a first time period a flow of the cell culture mixture from the fermentor into the interior of the centrifugal separator,
- separating continuously during the first time period the flow of the cell culture mixture in the centrifugal separator into a light phase and a heavy phase, the light phase forming a maximum of 5 %V/V of the flow of the cell culture mixture, and
- returning the heavy phase to the fermentor during the first time period simultaneously with the step of separating

According to a further aspect there is provided a use of a centrifugal separator comprising a surface enlarging insert for separating a cell containing heavy phase form a cell culture mixture during a fermentation process operated in perfusion mode.

More specifically, the use may relate to a use of a disc stack centrifugal separator for separating a cell containing heavy phase and a cell debris containing light phase from a cell culture mixture during a fermentation process operated in perfusion mode

The centrifugal separator may thus be used before and end of the fermentation step of the fermentation process. The fermentation process may be for producing a biomolecule.

According to a further aspect, there is provided a computer program comprising instructions which, when the program is executed by a computer, causes the computer to carry out a method 100 according to any one of aspects and/or embodiments discussed herein.

One skilled in the art will appreciate that the method 100 for operating a bioprocessing system may be implemented by programmed instructions. These programmed instructions are typically constituted by a computer program, which, when it is executed in a computer or calculation unit 52, ensures that the computer or calculation unit 52 carries out the desired control, such as one or more of the method steps 102 -152 according to the invention. The computer program is usually part of a computer-readable storage medium which comprises a suitable digital storage medium on which the computer program is stored.

**Fig. 6** illustrates embodiments of a computer-readable storage medium 99 comprising instructions which, when executed by a computer or calculation unit 52, cause the computer or calculation unit 52 to carry out one or more of the steps of the method 100 according to any one of aspects and/or embodiments discussed herein.

The computer-readable storage medium 99 may be provided for instance in the form of a data carrier carrying computer program code for performing at least some of the steps 102 - 152 according to some embodiments when being loaded into the one or more calculation units 52. The data carrier may be, e.g. a ROM (read-only memory), a PROM (programable read-only memory), an EPROM (erasable PROM), a flash memory, an EEPROM (electrically erasable PROM), a hard disc, a CD ROM disc, a memory stick, an optical storage device, a magnetic storage device or any other appropriate medium such as a disk or tape that may hold machine readable data in a non-transitory manner. The computer-readable storage medium may furthermore be provided as computer program code on a server and may be downloaded to the calculation unit 52 remotely, e.g., over an Internet or an intranet connection, or via other wired or wireless communication systems.

The computer-readable storage medium 99 shown in **Fig. 6** is a nonlimiting example in the form of a USB memory stick.

It is to be understood that the foregoing is illustrative of various example embodiments and that the invention is defined only by the appended claims. A person skilled in the art will realize that the example embodiments may be modified, and that different features of the example embodiments may be combined to create embodiments other than those described herein, without departing from the scope of the invention, as defined by the appended claims.

The following is a non-limiting and itemized listing of embodiments of the present disclosure, presented for the purpose of describing various features and combinations provided by the invention in certain of its aspects.

ITEMIZED LISTING OF EMBODIMENTS

1. A method (100) for operating a bioprocessing system (2), comprising steps of:

- producing (102) a cell culture mixture in a fermentor (4),
- conducting (104) a flow of cell culture mixture from the fermentor (4) into an interior of a centrifugal separator (6) comprising a surface enlarging insert, simultaneously with the step of producing (102) the cell culture mixture, and
- returning (106) continuously from the interior of the centrifugal separator (6) a flow of liquid to the fermentor (4), simultaneously with the steps of producing (102) the cell culture mixture and conducting (104) the flow of cell culture mixture.

2. The method (100) for operating a bioprocessing system (2) according to item 1, comprising during a first time period a step of:

- separating (107) the flow of cell culture mixture into a light phase and a heavy phase in the centrifugal separator (6), and wherein the step of returning (106) continuously from the interior of the centrifugal separator (6) a flow of liquid to the fermentor (4) comprises during the first time period:
- returning (108) the heavy phase separated in the centrifugal separator (6) to the fermentor (4) as the flow of liquid to the fermentor (4).

3. The method (100) according to item 2, wherein in the step of separating (107) the flow of cell culture mixture into a light phase and a heavy phase, a flow of the separated light phase forms a maximum of 5 %V/V of the flow of cell culture mixture.

4. The method (100) according to item 2 or 3, comprising during the first time period a step of:

- conducting (112) the light phase separated in the centrifugal separator (6) to a receiving container (8).

5. The method (100) according to any one of items 2- 4, wherein during the first time period, the heavy phase comprises a growth medium and whole cells and the light phase comprises the growth medium and debris from the cell culture mixture of a particle size smaller than whole cells and is substantially free of whole cells.

6. The method (100) according to any one of items 2 - 5, wherein during the first time period, the flow of the cell culture mixture is conducted at a flowrate, q, wherein the centrifugal separator (6) is operated at an Area Equivalent, Ae, such that at the flowrate, q, whole cells having a diameter d are separated from the cell culture mixture.

7. The method (100) according to any one of items 2 - 6, wherein preceding the first time period, the method comprises a step of:

- priming (114) the centrifugal separator (6) with a liquid other than cell culture mixture, such as a growth medium.

8. The method (100) for operating a bioprocessing system (2) according to any one of items 2 - 7, wherein the step of returning (106) continuously from the interior of the centrifugal separator (6) a flow of liquid to the fermentor (4) comprises during a second time period a step of:

- returning (110) the flow of cell culture mixture from the interior of the centrifugal separator (6) unseparated to the fermentor (4) as the flow of liquid to the fermentor (4).

9. The method (100) according to item 8, wherein during the second time period during the step of returning (110)

the flow of cell culture mixture, a rotor of the centrifugal separator (6) is standing still or rotating at a lower rotational speed than during the first time period during the step of separating (107) the flow of cell culture mixture, or rotating in a rotational direction opposite to a rotational direction during the first time period during the step of separating (107) the flow of cell culture mixture.

10. The method (100) according to item 8 or 9, wherein the first and second time periods are alternated.

11. The method (100) according to any one of items 8 - 10, wherein during the first and second time periods, the flow of the cell culture mixture is conducted at substantially a same flowrate, q, into the interior of the centrifugal separator (6).

12. The method (100) according to any one of the preceding items, comprising during a third time period steps of:

- stopping (115) the step of returning (106) continuously from the interior of the centrifugal separator (6) a flow of liquid to the fermentor (4),
- conducting (116) a flow of the cell culture mixture from the fermentor (4) to the centrifugal separator (6),
- separating (118) the flow of cell culture mixture in the centrifugal separator (6) into a light phase and a heavy phase, and
- conducting (120) the heavy phase separated in the step of separating (118) to a receiving vessel (18).

13. The method (100) according to item 12, comprising during the third time period a step of:

- conducting (122) the light phase separated in the step of separating (118) to a receiving container (8).

14 The method (100) according to item 12 or 13, wherein during the third time period, a rotor of the centrifugal separator (6) is rotating at a higher rotational speed than during the first time period.

15. The method (100) according to any one of the preceding items, comprising during a fourth time period steps of:

- stopping (115) the step of returning (106) continuously from the interior of the centrifugal separator (6) a flow of liquid to the fermentor (4),
- conducting (124) all of the cell culture mixture from the fermentor (4) to the centrifugal separator (6),
- separating (126) the cell culture mixture in the centrifugal separator (6) into a light phase and a heavy phase, the light phase forming 50 - 95 %V/V of the cell culture mixture, and
- conducting (130) the light phase separated in the centrifugal separator (6) to a receiving container (8).

16. The method (100) according to item 15, comprising during the fourth time period a step of:

- conducting (128) the heavy phase separated in the centrifugal separator (6) to a receiving vessel (18).

17. The method (100) according to any one of items 12 - 16, wherein subsequently to the third and/or fourth time period, the method (100) comprises steps of:

- diluting (132) the heavy phase in the receiving vessel (18) with a liquid to produce a diluted heavy phase,
- transferring (134) during a fifth time period the diluted heavy phase from the receiving vessel (18) to a centrifugal apparatus, such as the centrifugal separator (6),
- separating (136) during the fifth time period the diluted heavy phase in the centrifugal apparatus into a light phase and a further heavy phase,
- conducting (138) the light phase to the receiving container (8).

18. The method (100) according to item 17, wherein during the fifth period, the rotor of the centrifugal separator (6) is rotated at a higher rotational speed than during the first time period.

19. The method (100) according to any one of items 12 - 18, wherein subsequently to the third, fourth, and/or fifth time period, the method (100) comprises during a sixth time period steps of:

- flushing (140) the centrifugal separator (6) with a liquid, such as a growth medium,
- running (142) the centrifugal separator (6), and

- conducting (144) at least part of the liquid via a light phase outlet of the centrifugal separator (6) to the receiving container (8).

20. The method (100) according to item 19, wherein during the step of running (142) the centrifugal separator (6), the rotor of the centrifugal separator (6) is rotated at a higher rotational speed than during the first time period.

21. The method (100) according to any one of items 15 - 20, wherein subsequently to any one of the first to sixth time period, the method (100) comprises steps of:

- conducting (146) the light phase collected in the receiving container (8) to the centrifugal separator (6) or to a centrifugal apparatus (6'),
- separating (148) the light phase in the centrifugal separator (6) or the centrifugal apparatus (6') into a light fraction and a heavy fraction, the heavy fraction comprising remaining debris from the cell culture mixture, and
- transferring (150) the light fraction for further processing, such as extraction of an extracellular substance from the light fraction.

22. The method (100) according to item 23, wherein during the step of separating (148) the light phase in the centrifugal apparatus into a light fraction and a heavy fraction, the rotor of the centrifugal separator (6) is rotated at a higher rotational speed than during one or more of the first to sixth time period.

23. The method (100) according to any one of the preceding items, wherein the centrifugal separator (6) comprising a surface enlarging insert is a modular centrifugal separator (6) comprising a base unit and an exchangeable separation insert mounted in the base unit, and wherein the method (100) comprises a step of:

- exchanging a used exchangeable separation insert for an unused exchangeable separation insert prior to the step of (104) a flow of cell culture mixture from the fermentor (4).

24. A bioprocessing system (2) comprising a fermentor (4) for producing therein a cell culture mixture, a centrifugal separator (6) comprising a surface enlarging insert, a receiving container (8), a conduit system interconnecting the fermentor (4), the centrifugal separator (6), and the receiving container (8), flow control equipment arranged in the conduit system, and a control system configured for controlling the centrifugal separator (6) and at least part of the flow control equipment, wherein
the control system is configured to:

- conduct a flow of cell culture mixture from the fermentor (4) into an interior of the centrifugal separator (6), and simultaneously,
- continuously return from the interior of the centrifugal separator (6) a flow of liquid to the fermentor (4).

25. The system according to item 24, wherein the control system is configured during a first time period to:

- run the centrifugal separator (6) to separate the flow of cell culture mixture into a light phase and a heavy phase, and
- continuously return from the interior of the centrifugal separator (6) the heavy phase separated in the centrifugal separator (6), as the flow of liquid to the fermentor (4).

26. The system according to item 25, wherein the conduit system interconnects the centrifugal separator (6) with the receiving container (8), and wherein the control system further is configured during the first time period to:

- conduct the light phase separated in the centrifugal separator (6) to the receiving container (8).

27. The system according to any one of items 24 - 26, wherein the control system is configured during a second time period to:

- continuously return from the interior of the centrifugal separator (6) the cell culture mixture unseparated, as the flow of liquid to the fermentor (4).

28. The system according to item 27, wherein the control system further is configured during the second time period to, maintain a rotor of the centrifugal separator (6) standing still, or rotate the rotor at a lower rotational speed than during the first time period, or rotate the rotor in a rotational direction opposite to a rotational direction during the first time period.

29. The system according to any one of items 25 - 28, wherein the control system is configured to alternate the first and second time periods.

30. The system according to any one of items 24- 29, comprising a receiving vessel (18), the conduit system interconnecting the centrifugal separator (6) with the receiving vessel (18), wherein the control system is configured during a third time period to:

- stop the continuous return from the interior of the centrifugal separator (6) of the flow of liquid to the fermentor (4),
- conduct a flow of the cell culture mixture from the fermentor (4) to the centrifugal separator (6),
- run the centrifugal separator (6) to separate the flow of cell culture mixture in the centrifugal separator (6) into a light phase and a heavy phase, and
- conduct the heavy phase separated in the centrifugal separator (6) to the receiving vessel (18).

31. The system according to item 30, wherein the control system is configured during the third time period to:

- conduct the light phase separated in the centrifugal separator (6) to the receiving container (8).

32. The system according to item 30 or 31, wherein the centrifugal separator (6) comprises a rotor, and wherein the control system is configured to rotate the rotor at a higher rotational speed during the third time period than during the first time period.

33. The system according to any one of items 24 - 32, wherein the control system is configured during a fourth time period to:

- stop the continuous return from the interior of the centrifugal separator (6) of the flow of liquid to the fermentor (4),
- conduct all of the cell culture mixture from the fermentor (4) to the centrifugal separator (6),
- run the centrifugal separator (6) to separate the cell culture mixture into a light phase and a heavy phase, and
- conduct the light phase separated in the centrifugal separator (6) to the receiving container (8).

34. The system according to item 33, wherein the control system is configured during the fourth time period to:

- conduct the heavy phase separated in the centrifugal separator (6) to the receiving vessel (18).

35. The system according to any one of items 25 - 34, optionally comprising a centrifugal apparatus and optionally the conduit system interconnecting the receiving container (8) with the centrifugal apparatus, wherein the control system is configured subsequently to any one of the first to sixth time period to:

- conduct the light phase collected in the receiving container (8) to the centrifugal separator (6) or the centrifugal apparatus,
- run the centrifugal separator (6) or the centrifugal apparatus to separate the light phase into a light fraction and a heavy fraction, and
- transfer the light fraction from the centrifugal separator (6) or the centrifugal apparatus.

36. The system according to any one of items 25 - 35, wherein the control system is configured during the first and second time periods to provide a flow of the cell culture mixture at substantially a same flowrate, q, into the interior of the centrifugal separator (6).

37. The system according to any one of items 24 - 36, wherein the centrifugal separator (6) comprising a surface enlarging insert is a modular centrifugal separator (6) comprising a base unit and an exchangeable separation insert mounted in the base unit.

38. The system according to any one of items 24 - 37, wherein the conduit system comprises a first conduit section extending from the fermentor (4) to an inlet of the centrifugal separator (6), a second conduit section extending from a heavy phase outlet of the centrifugal separator (6) to the fermentor (4), a third conduit section extending from a light phase outlet of the centrifugal separator (6) to the receiving container (8), and wherein the flow control equipment comprises a feed pump arranged in the first conduit section, a heavy phase pump arranged in the second conduit section, a light phase pump arranged in the third conduit section, a first valve arrangement arranged at the first conduit section, a second valve arrangement arranged at the second conduit section, and a third valve arrangement arranged at the third conduit section.

39. The system according to item 38, wherein the feed pump is a centrifugal pump, and each of the heavy phase pump and the light phase pump is a peristaltic pump.

40. The system according to any one of items 25 - 37 and item 38 or 39, wherein the conduit system comprises a fourth conduit section extending to the first conduit section, the fourth conduit section being configured for supplying a liquid other than the cell culture mixture to the inlet of the centrifugal separator (6), and wherein the control system is configured prior to the first time period to:

   run the feed pump, the heavy phase pump, and the light phase pump,

   - provide a liquid passage from the fourth conduit section to the first conduit section and the centrifugal separator (6) utilising the first valve arrangement,
   - provide a diverting passage from the second conduit section utilising the second valve arrangement, and
   - provide a diverting passage from the third conduit utilising the third valve arrangement,

   such that the liquid other than cell culture mixture is transferred via the fourth and first conduit sections to the inlet and the interior of the centrifugal separator (6) and out of centrifugal separator (6) via the heavy phase outlet and the light phase outlet and being diverted from the second and third conduit sections before reaching the fermentor (4) and the receiving container (8).

41. The system according any one of items 25 - 37 and any one of items 38 - 40, wherein the control system during the first time period is configured to:

   - run the feed pump, the heavy phase pump, and the light phase pump,
   - provide a liquid passage from the fermentor (4) to the first conduit section and the centrifugal separator (6) utilising the first valve arrangement,
   - provide a heavy phase passage via the second conduit section to the fermentor (4) utilising the second valve arrangement, and
   - provide a light phase passage via the third conduit to the receiving container (8) utilising the third valve arrangement.

42. The system according any one of items 27 - 37 and any one of items 38 - 41, wherein the control system is configured during the second time period to:

   - run the feed pump and the heavy phase pump,
   - maintain the first and second valve arrangements open, and
   - maintain the third valve arrangement closed, such as to provide a cell culture mixture passage from the fermentor (4) via the first conduit section to and through the centrifugal separator (6) and therefrom via the second conduit section returned back to the fermentor (4).

43. The system according to any one of items 30 - 32 or 34 and any one of items 38 - 42, wherein the conduit system comprises a fifth conduit section extending from the second conduit section to the receiving vessel (18), wherein the control system is configured during the third time period to:

   - run the feed pump and the heavy phase pump,
   - run the centrifugal separator (6), and
   - provide a heavy phase passage from the heavy phase outlet to the receiving vessel (18) utilising the second valve arrangement and the fifth conduit section, such that the cell culture mixture is transferred from the fermentor (4) via the first conduit section to the centrifugal separator (6), separated in the centrifugal separator (6) into a light phase which is conducted via the third conduit section to the receiving container (8) and a heavy phase which is conducted via the first and fifth conduit sections to the receiving vessel (18).

44. The system according to any one of items 24 - 43, wherein the centrifugal separator (6) is arranged on the fermentor (4).

45. The system according to any one of items 24 - 44, wherein the control system is configured to control the fermentor (4), such as a pump of the fermentor (4), a valve of the fermentor (4), an air inlet to the fermentor (4), a stirring member arranged in the fermentor (4).

46. A computer program comprising instructions which, when the program is executed by a computer, cause the computer to carry out the method (100) according to any one of items 1 - 23.

47. A computer-readable storage medium comprising instructions which, when executed by a computer, cause the computer to carry out the method (100) according to any one of items 1 - 23.

**Claims**

1. A method (100) for operating a bioprocessing system (2), comprising steps of:

   - producing (102) a cell culture mixture in a fermentor (4),
   - conducting (104) a flow of cell culture mixture from the fermentor (4) into an interior of a centrifugal separator (6) comprising a surface enlarging insert, simultaneously with the step of producing (102) the cell culture mixture, and
   - returning (106) continuously from the interior of the centrifugal separator (6) a flow of liquid to the fermentor (4), simultaneously with the steps of producing (102) the cell culture mixture and conducting (104) the flow of cell culture mixture.

2. The method (100) for operating a bioprocessing system (2) according to claim 1, comprising during a first time period a step of:

   - separating (107) the flow of cell culture mixture into a light phase and a heavy phase in the centrifugal separator (6), and wherein the step of returning (106) continuously from the interior of the centrifugal separator (6) a flow of liquid to the fermentor (4) comprises during the first time period:
   - returning (108) the heavy phase separated in the centrifugal separator (6) to the fermentor (4) as the flow of liquid to the fermentor (4).

3. The method (100) according to claim 2, wherein in the step of separating (107) the flow of cell culture mixture into a light phase and a heavy phase, a flow of the separated light phase forms a maximum of 5 %V/V of the flow of cell culture mixture.

4. The method (100) according to claim 2 or 3, comprising during the first time period a step of:

   - conducting (112) the light phase separated in the centrifugal separator (6) to a receiving container (8).

5. The method (100) according to any one of claims 2-4, wherein during the first time period, the heavy phase comprises a growth medium and whole cells and the light phase comprises the growth medium and debris from the cell culture mixture of a particle size smaller than whole cells and is substantially free of whole cells.

6. The method (100) according to any one of claims 2 - 5, wherein preceding the first time period, the method comprises a step of:

   - priming (114) the centrifugal separator (6) with a liquid other than cell culture mixture, such as a growth medium.

7. The method (100) for operating a bioprocessing system (2) according to any one of claims 2 - 6, wherein the step of returning (106) continuously from the interior of the centrifugal separator (6) a flow of liquid to the fermentor (4) comprises during a second time period a step of:

   - returning (110) the flow of cell culture mixture from the interior of the centrifugal separator (6) unseparated to the fermentor (4) as the flow of liquid to the fermentor (4).

8. The method (100) according to claim 7, wherein during the second time period during the step of returning (110) the flow of cell culture mixture, a rotor of the centrifugal separator (6) is standing still or rotating at a lower rotational speed than during the first time period during the step of separating (107) the flow of cell culture mixture, or rotating in a rotational direction opposite to a rotational direction during the first time period during the step of separating (107) the flow of cell culture mixture.

9. The method (100) according to any one of the preceding claims, comprising during a third time period steps of:

   - stopping (115) the step of returning (106) continuously from the interior of the centrifugal separator (6) a flow of liquid to the fermentor (4),
   - conducting (116) a flow of the cell culture mixture from the fermentor (4) to the centrifugal separator (6),
   - separating (118) the flow of cell culture mixture in the centrifugal separator (6) into a light phase and a heavy phase, and
   - conducting (120) the heavy phase separated in the step of separating (118) to a receiving vessel (18).

10. The method (100) according to claim 9, comprising during the third time period a step of:

   - conducting (122) the light phase separated in the step of separating (118) to a receiving container (8).

11. The method (100) according to any one of the preceding claims, comprising during a fourth time period steps of:

   - stopping (115) the step of returning (106) continuously from the interior of the centrifugal separator (6) a flow of liquid to the fermentor (4),
   - conducting (124) all of the cell culture mixture from the fermentor (4) to the centrifugal separator (6),
   - separating (126) the cell culture mixture in the centrifugal separator (6) into a light phase and a heavy phase, the light phase forming 50 - 95 %V/V of the cell culture mixture, and
   - conducting (130) the light phase separated in the centrifugal separator (6) to a receiving container (8).

12. The method (100) according to any one of claims -9 - 11, wherein subsequently to the third and/or fourth time period, the method (100) comprises steps of:

   - diluting (132) the heavy phase in the receiving vessel (18) with a liquid to produce a diluted heavy phase,
   - transferring (134) during a fifth time period the diluted heavy phase from the receiving vessel (18) to a centrifugal apparatus, such as the centrifugal separator (6),
   - separating (136) during the fifth time period the diluted heavy phase in the centrifugal apparatus into a light phase and a further heavy phase,
   - conducting (138) the light phase to the receiving container (8).

13. The method (100) according to any one of claims -9 - 12, wherein subsequently to the third, fourth, and/or fifth time period, the method (100) comprises during a sixth time period steps of:

   - flushing (140) the centrifugal separator (6) with a liquid, such as a growth medium,
   - running (142) the centrifugal separator (6), and
   - conducting (144) at least part of the liquid via a light phase outlet of the centrifugal separator (6) to the receiving container (8).

14. The method (100) according to any one of claims -11 - 13, wherein subsequently to any one of the first to sixth time period, the method (100) comprises steps of:

   - conducting (146) the light phase collected in the receiving container (8) to the centrifugal separator (6) or to a centrifugal apparatus (6'),
   - separating (148) the light phase in the centrifugal separator (6) or the centrifugal apparatus (6') into a light fraction and a heavy fraction, the heavy fraction comprising remaining debris from the cell culture mixture, and
   - transferring (150) the light fraction for further processing, such as extraction of an extracellular substance from the light fraction.

15. The method (100) according to any one of the preceding claims, wherein the centrifugal separator (6) comprising a surface enlarging insert is a modular centrifugal separator (6) comprising a base unit and an exchangeable separation insert mounted in the base unit, and wherein the method (100) comprises a step of:

- exchanging a used exchangeable separation insert for an unused exchangeable separation insert prior to the step of (104) a flow of cell culture mixture from the fermentor (4).

16. A bioprocessing system (2) comprising a fermentor (4) for producing therein a cell culture mixture, a centrifugal separator (6) comprising a surface enlarging insert, a receiving container (8), a conduit system interconnecting the fermentor (4), the centrifugal separator (6), and the receiving container (8), flow control equipment arranged in the conduit system, and a control system configured for controlling the centrifugal separator (6) and at least part of the flow control equipment, wherein
the control system is configured to:

- conduct a flow of cell culture mixture from the fermentor (4) into an interior of the centrifugal separator (6), and simultaneously,
- continuously return from the interior of the centrifugal separator (6) a flow of liquid to the fermentor (4).

**Fig. 1**

**Fig. 2**

**Fig. 3**

16

56    6    12    14

52

54

**Fig. 4**

99

**Fig. 6**

Volume
Fraction
%

0.1    1    10    μm

**Fig. 7**

100

**Fig. 5**

Europäisches
Patentamt

European
Patent Office

Office européen
des brevets

# EUROPEAN SEARCH REPORT

Application Number

EP 20 21 4285

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/251716 A1 (LAUSTSEN MADS [DK] ET AL) 6 September 2018 (2018-09-06) * paragraphs [0009], [0053], [0054], [0057], [0096] - [0086]; figure 1 * | 1-16 | INV. C12M1/00 C12M1/26 |
| X | WO 2019/226618 A1 (NANTKWEST INC [US]) 28 November 2019 (2019-11-28) * paragraphs [0053] - [0055], [0058], [0059]; figure 2 * | 1-16 | |
| A | US 2016/137976 A1 (MEHTA SUNIL [US] ET AL) 19 May 2016 (2016-05-19) * paragraphs [0106] - [0111]; figures 12, 13 * | 1,7,8, 12-14 | |
| A,D | EP 3 666 394 A1 (ALFA LAVAL CORP AB [SE]) 17 June 2020 (2020-06-17) * paragraphs [0032] - [0034], [0064], [0074], [0089] - [0101]; figures 1,3-5 * | 1-16 | |

| | | | TECHNICAL FIELDS SEARCHED (IPC) |
|---|---|---|---|
| | | | C12M |

The present search report has been drawn up for all claims

| Place of search | Date of completion of the search | Examiner |
|---|---|---|
| Berlin | 5 May 2021 | Pantelidis, D |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.................................................................
& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 20 21 4285

This annex lists the patent family members relating to the patent documents cited in the above-mentioned European search report.
The members are as contained in the European Patent Office EDP file on
The European Patent Office is in no way liable for these particulars which are merely given for the purpose of information.

05-05-2021

| Patent document cited in search report | | Publication date | Patent family member(s) | | Publication date |
|---|---|---|---|---|---|
| US 2018251716 | A1 | 06-09-2018 | EP | 3071682 A1 | 28-09-2016 |
| | | | US | 2016298072 A1 | 13-10-2016 |
| | | | US | 2018251716 A1 | 06-09-2018 |
| | | | WO | 2015075070 A1 | 28-05-2015 |
| WO 2019226618 | A1 | 28-11-2019 | EP | 3797149 A1 | 31-03-2021 |
| | | | WO | 2019226618 A1 | 28-11-2019 |
| US 2016137976 | A1 | 19-05-2016 | AU | 2009271588 A1 | 21-01-2010 |
| | | | AU | 2015200830 A1 | 12-03-2015 |
| | | | CA | 2730528 A1 | 21-01-2010 |
| | | | EP | 2310486 A2 | 20-04-2011 |
| | | | JP | 5746025 B2 | 08-07-2015 |
| | | | JP | 6234958 B2 | 22-11-2017 |
| | | | JP | 2011528225 A | 17-11-2011 |
| | | | JP | 2015180204 A | 15-10-2015 |
| | | | KR | 20110031383 A | 25-03-2011 |
| | | | SG | 168174 A1 | 28-02-2011 |
| | | | SG | 192529 A1 | 30-08-2013 |
| | | | US | 2011207222 A1 | 25-08-2011 |
| | | | US | 2011207225 A1 | 25-08-2011 |
| | | | US | 2016137976 A1 | 19-05-2016 |
| | | | WO | 2010008563 A2 | 21-01-2010 |
| | | | WO | 2010008579 A2 | 21-01-2010 |
| EP 3666394 | A1 | 17-06-2020 | EP | 3666394 A1 | 17-06-2020 |
| | | | WO | 2020120359 A1 | 18-06-2020 |

EPO FORM P0459

For more details about this annex : see Official Journal of the European Patent Office, No. 12/82

**EP 4 015 614 A1**

**REFERENCES CITED IN THE DESCRIPTION**

**Patent documents cited in the description**

- WO 2017025210 A
- EP 3666394 A